# EUROPEAN PATENT APPLICATION

(11) **EP 4 075 141 A2**
(43) Date of publication of application: **19.10.2022**
(21) Application number: 20896087.2
(22) Date of filing: 04.12.2020
(51) Int. Cl.: G01N 33/68

(54) **METHOD FOR PREDICTING REACTIVITY TO DRUG TARGETING BCL2 FAMILY PROTEIN**

(30) Priority: 06.12.2019 KR 20190162071; 23.11.2020 KR 20200157961
(71) Applicant: Proteina Co., Ltd., Seoul 08826 (KR)
(72) Inventor: YOON, Tae-Young, Seoul 06280 (KR); LEE, Hongwon, Seoul 06966 (KR); CHOI, Byoungsan, Seoul 03692 (KR); CHA, Minkwon, Seoul 08792 (KR); HONG, Saem, Seoul 06659 (KR); LEE, Yunseo, Paju-si, Gyeonggi-do 10871 (KR); CHUN, Changju, Seoul 08832 (KR); KOH, Youngil, Seoul 06082 (KR); YOON, Sung-Soo, Seoul 06288 (KR)
(74) Representative: Bates, Philip Ian
(86) International application number: PCT/KR2020/017691
(87) International publication number: WO 2021/112635

(57) **Abstract**

Provided are a method for predicting reactivity to a drug targeting a BCL2 family protein, and a method for selecting a subject suitable for treatment with a drug targeting a BCL2 family protein.

## Description

### [Technical Field]

Provided are a method for prediction of a response to a BCL2 family protein-targeting drug and a method for selection of a subject suitable for treatment with a BCL2 family protein-targeting drug.

### [Background Art]

A BCL2 (B-cell lymphoma 2) family protein regulates apoptosis through apoptosis induction (pro-apoptotic activity) or inhibition (anti-apoptotic activity).

BCL2 family proteins are responsible for causing various cancers including melanoma, breast cancer, prostate cancer, chronic lymphocytic leukemia, and lung cancer, and known as factors that induce resistance to cancer therapy. It has been also suggested that BCL2 family proteins are likely to cause schizophrenia and autoimmune disease. For these reasons, BCL2 family proteins may be promising targets for anticancer agents, and various inhibitors targeting BCL2 family proteins have been developed.

In order to effectively employ BCL2 family protein-targeting drugs as cancer therapeutic drugs, there is a need for the development of a means for quantitatively analyzing apoptosis regulation signals induced by BCL2 family proteins.

### [Disclosure]

### [Technical Problem]

Provided is a personalized therapeutic technique in which a protein interaction that mediates signal transduction is quantitatively analyzed, whereby a person could be treated for cancer (for example, blood cancer) with a BCL2 family protein-targeting drug.

An aspect provides a method for prediction of a response of a subject to a BCL2 family protein-targeting drug or for prediction of a medicinal efficacy of a BCL2 family protein-targeting drug in a subject or a method for providing information for the prediction.

Another aspect provides a method for selection (identification) of a subject that is responsive to a BCL2 family protein-targeting drug, in which a BCL2 family protein-targeting drug exhibits medicinal efficacy, or to which a BCL2 family protein-targeting drug is suitably applicable, or a method for provision of information for the selection (identification).

The method may further comprise a step of administering the drug to a subject in which the drug is responsive or exhibits medicinal efficacy, or to a subject which is suitable for treatment with the drug.

Another aspect provides a composition comprising a BCL2 family protein-targeting drug for cancer therapy for a subject which is responsive to the BCL2 family protein-targeting drug, in which the BCL2 family protein-targeting drug exhibits medicinal efficacy, or to a subject which the BCL2 family protein-targeting drug or treatment therewith is suitably applicable.

Another aspect provides a method for treatment of cancer, the method comprising a step of administering a pharmaceutically effective amount of a BCL2 family protein-targeting drug confirmed by the said method to a subject, which is responsive to the BCL2 family protein-targeting drug, in which the BCL2 family protein-targeting drug exhibits medicinal efficacy, or to which the BCL2 family protein-targeting drug or treatment therewith is suitably applicable.

Provided is a method for treatment of cancer, the method comprising a step of administering a pharmaceutically effective amount of the BCL2 family protein-targeting drug.

The BCL2 family protein-targeting drug may target a BCL2 family protein (e.g., a BCL2 protein inhibitor or a MCL1 protein inhibitor).

### [Technical Solution]

The method for prediction of a response of a subject to a BCL2 family protein-targeting drug or for prediction of medicinal efficacy of a BCL2 family protein-targeting drug in a subject or the method for providing information for the prediction; and/or the method for selection (identification) of a subject that is responsive to a BCL2 family protein-targeting drug, in which a BCL2 family protein-targeting drug exhibits medicinal efficacy, or to which a BCL2 family protein-targeting drug is suitably applicable, or the method for provision of information for the selection (identification), all provided herein, comprise the steps of: (a1) measuring interaction between apoptosis inhibiting (anti-apoptotic) protein and an apoptosis inducing (pro-apoptotic) protein within a sample (e.g., the following steps (i) and/or (ii)); and/or (a2) measuring a level of the apoptosis inducing (pro-apoptotic) protein within a sample and/or the apoptosis inhibiting (anti-apoptotic) protein (e.g., the following step (iii)).

In an embodiment, the method, provided herein, may comprise at least one (e.g., one, two, or three) selected from the following steps:
(i) a step of measuring interaction between a first protein within a sample and a protein interacting with the first protein, wherein the protein interacting with the first protein is externally provided (first protein-protein interaction measuring step);
(ii) a step of measuring interaction between a first protein endogenous to a sample and a protein interacting with the first protein, wherein the protein interacting with the first protein is endogenous to the sample (second protein-protein interaction measuring step); and
(iii) a step of measuring a level of the first protein, the protein interacting with the first protein, or both thereof in the sample.

In an embodiment, the method, provided herein, may comprise step (i), step (ii), or step (iii). In another embodiment, the method, provided herein, may comprise a combination of step (i) and step (ii), a combination of step (i) and step (iii), a combination of step (ii) and step (iii), or a combination of step (i), step (ii), and step (iii).

The first protein and the protein interacting with the first protein (hereinafter, "protein interacting with the first protein" is also referred to as "first protein-interacting protein") may be selected from anti-apoptotic subtype proteins and cell survival inducing (pro-survival) subtype proteins.

In an embodiment, the first protein in steps (i) to (iii) may be at least one selected from "pro-survival or anti-apoptotic subtype proteins" (hereinafter, referred to as "anti-apoptotic protein") of BCL2 family proteins. For example, the first proteins in steps (i) to (iii), which may be the same or different, may each be independently at least one species (e.g., one, two, or three species) selected from the group consisting of BCL2, BCLxL, BCLw, BFL1, and MCL1. The protein interacting with the first protein in steps (i) to (iii) may be at least one selected from pro-apoptotic subtype proteins (hereinafter, "pro-apoptotic subtype protein" is referred to as "pro-apoptotic protein") in the BCL2 family proteins. In an embodiment, the first protein-interacting proteins in steps (i) to (iii), which may be the same or different, may each be independently at least one selected from the group consisting of a BH3 subtype (BH3-only) protein (second protein) and an effector subtype protein (third protein) in the pro-apoptotic proteins. For example, the second protein may be at least one species (e.g., one, two, or three species) selected from the group consisting of BAD, BIM, PUMA, BMF, BID, tBID, BIK, HRK, and NOXA, and the third protein may be at least one species (e.g., one, or two species) selected from the group consisting of BAX, BOK, and BAK. In an embodiment, the first protein-interacting protein may be at least one species (e.g., one, two, three, four, or five species) selected from the group consisting of BAD, BIM, PUMA, BMF, BID, tBID, BIK, HRK, NOXA, BAX. BOK, and BAK. When the method comprises two or more of steps (i) to (iii), the first protein-interacting proteins in the respective steps may be the same or different.

In another embodiment, the first protein in steps (i) to (iii) may be at least one selected from the pro-apoptotic proteins in BCL2 family proteins. In an embodiment, the first proteins in steps (i) to (iii), which may be the same or different, may be at least one species selected from the group consisting of a BH3 subtype (BH3-only) protein and an effector subtype protein in the pro-apoptotic proteins of the BCL2 family proteins. By way of example, the BH3 subtype (BH3-only) protein is at least one species (e.g., one, two, or three species) selected from the group consisting of BAD, BIM, PUMA, BMF, tBID, BIK, HRK, and NOXA, and the effector subtype protein may be at least one species (e.g., one or two species) selected from the group consisting of BAX, BOK, and BAK. The first protein-interacting protein in steps (i) to (iii) may be at least one species selected from anti-apoptotic proteins in BCL2 family proteins. For example, the first protein-interacting proteins in steps (i) to (iii), which may be the same or different, may each be independently at least one (e.g., one, two, or three species) selected from the group consisting of BCL2, BCLxL, BCLw, BFL-1, and MCL1.

When the method comprises two or more of steps (i) to (iii), the first proteins and the first protein-interacting proteins in the respective steps may be the same or different.

As used herein, the term "sample" (biological sample) is intended to encompass a cell or cell lysate isolated from a subject (e.g., human). As used herein, the term "subject" refers to an animal in need of treating a disease (e.g., cancer) and means a subject to which the BCL2 family protein-targeting drug is to be administered, a subject to which the treatment with the BCL2 family protein-targeting drug is to be applied, or a subject in which the administration of the BCL2 family protein-targeting drug and the treatment with the BCL2 family protein-targeting drug needs to be identified for medical efficacy (drug response). In an embodiment, the subject may be a cancer patient (e.g., a human) and the cell may be a cancer cell isolated from the subject. For example, the subject may be a human patient with blood cancer and the sample may be at least one selected from the group consisting of leukocytes isolated from blood cancer patient (for example, granular leukocytes, lymphocytes, and/or monocytes (e.g., peripheral blood mononuclear cells (PBMC), etc.) and so on), myelocytes (e.g., hematopoietic stem cells, etc.), blood, bone marrow, and buffy coat, each containing the cells, and lysates thereof. The sample obtained from a subject to be selected or identified in the method provided herein may be expressed as "test sample" to discriminate from a control sample for comparison (for example, a positive control sample or a negative control sample used to select a criterion value for comparison as described later).

As used herein, the term "externally provided protein" (e.g., first protein-interacting protein) refers to a protein that is not endogenous to a sample, but is added externally (exogenous) to the sample. In an embodiment, the externally provided protein may be labeled with a typical probe (e.g., fluorescent substance, etc.), but without limitations thereto.

In an embodiment, step (i) may comprise measuring interaction between a BCL2 family protein (first protein) within a sample and an externally provided (exogenous) BCL2 family protein (a first protein-interacting protein) (first protein-protein interaction measuring step), wherein the exogenous first protein-interacting protein labeled with a probe is brought into contact with the first protein immobilized to a substrate (protein in the sample), followed by measuring protein-protein interaction between the first protein (protein in the sample) and the protein interacting therewith (exogenous protein) (e.g., measuring a level of a complex formed between the first protein and the first protein-interacting protein).

In an embodiment, step (ii) may comprise measuring interaction between BCL2 family proteins endogenous to a sample (second protein-protein interaction measuring step), wherein the sample containing a first protein is brought into contact with a substrate including a substance that can bind to the first protein (e.g., an antibody to the first protein, etc.), followed by measuring protein-protein interaction between the first protein (protein endogenous to the sample) and the first protein-interacting protein (protein endogenous to the sample) (e.g., measuring a level of a complex formed between the first protein and the first protein-interacting protein).

In an embodiment, the level of a complex formed between the first protein and the protein interacting therewith in steps (i) and/or (ii) may be measured from the signal intensity generated by the probe conjugated to the first protein-interacting protein (step (i)) or to the substance binding to the first protein-interacting protein (step (ii)), but without limitations thereto.

In an embodiment, step (iii) may include a step of measuring a total expression level of either or both of the first protein and the first protein-interacting protein in the sample. The level in a sample may be an expression level of proteins (e.g., protein concentration), an expression level of activated proteins (e.g., activated protein concentration), an expression level of phosphorylated proteins (e.g., phosphorylated protein concentration), or a combination thereof in a sample.

In an embodiment, step (i) (first protein-protein interaction measuring step) may comprise at least one (e.g., 1, 2, 3, 4, 5, 6, 7, 8, 9, 10, 11, or 12) selected from the following sub-steps of:
(i-1) measuring protein-protein interaction between BCL2 protein within a sample and externally provided BAD protein;
(i-2) measuring protein-protein interaction between BCL2 protein within a sample and externally provided BIM protein;
(i-3) measuring protein-protein interaction between BCL2 protein within a sample and externally provided BAX protein;
(i-4) measuring protein-protein interaction between BCL2 protein within a sample and externally provided BAK protein;
(i-5) measuring protein-protein interaction between BCLxl protein within a sample and externally provided BAD protein;
(i-6) measuring protein-protein interaction between BCLxl protein within a sample and externally provided BIM protein;
(i-7) measuring protein-protein interaction between BCLxl protein within a sample and externally provided BAX protein;
(i-8) measuring protein-protein interaction between BCLxl protein within a sample and externally provided BAK protein;
(i-9) measuring protein-protein interaction between MCL1 protein within a sample and externally provided NOXA protein;
(i-10) measuring protein-protein interaction between MCL1 protein within a sample and externally provided BIM protein;
(i-11) measuring protein-protein interaction between MCL1 protein within a sample and externally provided BAX protein; and
(i-12) measuring protein-protein interaction between MCL1 protein within a sample and externally provided BAK protein.

In an embodiment, each of the sub-steps of measuring protein-protein interaction in step (i) (first protein-protein interaction measuring step) may be carried out by or may comprise:
(1) providing the sample to a substrate including a substance binding to the first protein to immobilize the first protein to the substrate;
(2-1) externally providing a probe-labeled protein interacting with the first protein to the substrate to allow a reaction with the first protein on the substrate; and
(3) measuring a signal generated by the probe within a near field region on the surface of the substrate using a total internal reflection fluorescence (TIRF) microscope.

In the first protein-protein interaction measuring step, the externally provided protein (first protein-interacting protein) may be one labeled (conjugated) with a probe. In the first protein-protein interaction measuring step, the measurement of protein-protein interaction may be carried out by measuring a level of a complex formed between the first protein (protein endogenous to a sample) and the protein interacting therewith (exogenous protein). In an embodiment, the level of the complex may be measured on the basis of the signal generated by the probe attached to the first protein-interacting protein.

In an embodiment, step (ii) (second protein-protein interaction measuring step) may comprise at least one (e.g., 1, 2, 3, 4, 5, 6, 7, 8, 9, 10, or 11) selected from the following sub-steps of:
(ii-1) measuring protein-protein interaction (e.g., a level of a complex thus formed) between BCL2 protein within a sample and BIM protein within a sample;
(ii-2) measuring protein-protein interaction (e.g., a level of a complex thus formed) between BCL2 protein within a sample and BAX protein within a sample;
(ii-3) measuring protein-protein interaction (e.g., a level of a complex thus formed) between BCL2 protein within a sample and BAK protein within a sample;
(ii-4) measuring protein-protein interaction (e.g., a level of a complex thus formed) between BCLxl protein within a sample and BAD protein within a sample;
(ii-5) measuring protein-protein interaction (e.g., a level of a complex thus formed) between BCLxl protein within a sample and BIM protein within a sample;
(ii-6) measuring protein-protein interaction (e.g., a level of a complex thus formed) between BCLxl protein within a sample and BAX protein within a sample;
(ii-7) measuring protein-protein interaction (e.g., a level of a complex thus formed) between BCLxl protein within a sample and BAK protein within a sample;
(ii-8) measuring protein-protein interaction (e.g., a level of a complex thus formed) between MCL1 protein within a sample and NOXA protein within a sample;
(ii-9) measuring protein-protein interaction (e.g., a level of a complex thus formed) between MCL1 protein within a sample and BIM protein within a sample;
(ii-10) measuring protein-protein interaction (e.g., a level of a complex thus formed) between MCL1 protein within a sample and BAX protein within a sample; and
(ii-11) measuring protein-protein interaction (e.g., a level of a complex thus formed) between MCL1 protein within a sample and BAK protein within a sample.

In an embodiment, each of the sub-steps of measuring protein-protein interaction (e.g., a level of a complex thus formed) in step (ii) (second protein-protein interaction measuring step) may be carried out by or may comprise:
(1) providing (adding) a sample to a substrate including a substance binding to the first protein to immobilize the first protein to the substrate;
(2-2) providing the substrate with a probe-labeled substance that bind to the first protein-interacting protein to allow a reaction with the first protein-interacting protein in a complex formed between the first protein (endogenous to the sample) and the first protein-interacting protein (endogenous to the sample);
(3) measuring a signal generated by the probe within a near field region on the surface of the substrate using a total internal reflection fluorescence (TIRF) microscope.

In another embodiment, each of the sub-steps of measuring protein-protein interaction (e.g., a level of a complex thus formed) in step (ii) (second protein-protein interaction measuring step) may be carried out by or may comprise:
(1) providing (adding) a sample to a substrate including a substance binding to the first protein-interacting protein to immobilize the first protein-interacting protein to the substrate;
(2-2) providing the substrate with a probe-labeled substance that bind to the first protein to allow a reaction with the first protein in a complex formed between the first protein (endogenous to the sample) and the first protein-interacting protein (endogenous to the sample); and
(3) measuring a signal generated by the probe within a near field region on the surface of the substrate using a total internal reflection fluorescence (TIRF) microscope.

In the second protein-protein interaction measuring step, the complex is endogenously formed between the first protein and the first protein-interacting protein, both endogenous to the sample. In the second protein-protein interaction measuring step, the measurement of protein-protein interaction may be carried out by measuring a level of a complex formed between the first protein (endogenous to the sample) and the protein interacting therewith (endogenous to the sample). In an embodiment, the level of a complex may be measured on the basis of a signal generated by the probe conjugated to a substance that binds to the first protein-interacting protein or the first protein.

In an embodiment, step (iii) may comprise at least one of the following sub-steps of:
(iii-1) measuring a level of BCL2 protein within the sample;
(iii-2) measuring a level of BCLxl protein within the sample;
(iii-3) measuring a level of MCL1 protein within the sample;
(iii-4) measuring a level of BAX protein within the sample; and
(iii-5) measuring a level of BAK protein within the sample.

Each of the level measurement sub-steps in step (iii) may be carried out by or may comprise:
- providing a sample to a substrate including either or both of a substance binding to the first protein (a capturing substance for immobilizing the first protein to a substrate) and a substance binding to the first protein-interacting protein (a capturing substance for immobilizing the first protein-interacting protein) to immobilize either or both of the first protein and the first protein-interacting protein to the substrate;
- providing a probe-labeled substance binding to the first protein (detector for detecting the first protein in the case where the first protein is immobilized to the substrate), a probe-labeled substance binding to the first protein-interacting protein (detector for detecting the first protein-interacting protein in the case where the first protein-interacting protein is immobilized to the substrate), or both of them (in the case where the first protein and the first protein-interacting protein are both immobilized to the substrate) to the substrate to allow a reaction; and
- measuring a signal generated by the probe within a near field region on the surface of the substrate using a total internal reflection fluorescence (TIRF) microscope.

The capturing substance and the detector, which bind to different sites of the same target protein, may each be independently selected from any binding substances (e.g., antibodies) the binding site of which completely does not overlap with each other.

The substance binding to the first protein binding substance or the first protein-interacting protein, used in in step (ii) and/or (iii), is a binder that is coupled to the first protein or the first protein-interacting protein (e.g., antibodies, antigen-binding fragments, antibody analogs, aptamers, small molecule compounds, and so on) and may be labeled with a probe. In this context, the sites of the first protein-interacting protein at which the binding substance and the first protein bind to the first protein-interacting protein, respectively, may be different from each other.

In one embodiment, the substance binding to the first protein for immobilizing (pulling-down) the first protein to a substrate in step (i), (ii), or (iii), the substance binding to the first protein-interacting protein for detecting a complex formed between the first protein and the first protein-interacting protein in step (ii), and/or the substance binding to the first protein or the first protein-interacting protein for detecting the first protein or the first protein-interacting protein in step (iii) may be at least one selected from typically available antibodies to the target proteins. When two or more of steps (i) to (iii) are carried out and the same first protein and/or the same first protein-interacting protein is commonly used in two of the steps, the binding substances used in the respective steps to immobilize (pull down) and/or detect the same protein may be the same and different.

Substances (e.g., antibodies) binding to the first protein and the first protein-interacting protein may be illustrated as follows:

### Anti-apoptotic protein

BCL2: ab32124, ab182858, ab238041, ab692 (all from Abcam), 3498S, 15071S, #4223 (all from CST), BSM-52304R, BSM-10846M, BSM-33411M, BSM-52022M (all from Bioss), NB100-56098 (Novus), 12789-1-AP (Proteintech), LS-C159170-400 (LSBio), 13-8800 (Thermo Fisher Scientific), 610538 (BD), sc-509, sc-23960, sc-7382 (all from Santa Cruz), TA806591, CF806589, UM800117, CF806639 (all from OriGene), and so on;
BCL-XL: 2764S, 2762S, 2870, 3498S (all from CST), ab77571, ab59348, ab32370, ab178844 (all from Abcam), MA5-11950, MA5-28637 (all from Invitrogen), MA5-15142 (Thermo Fisher Scientific), BSM-51229M (Bioss), sc-56021, sc-136207, sc-271121, sc-70417, sc-23958 (all from Santa Cruz), ALX-804-660-C100 (Enzo), 66020-1-IG (Proteintech), NBP2-25240, NBP2-32810, NBP2-44416, NBP1-47665 (all from Novus), TA506188 (OriGene), H00000598-M01A (Abnova), and so on;
MCL1: ab32087 (Abcam), 5453S, 39224S, #94296 (all from CST), MA5-32060, 14-6701-82, 14-9047-82, MA5-15236, 14-6701-82, 14-9047-82 (all from Invitrogen), SP2170P, TA500986, TA500990 (all from OriGene), 16225-1-AP (Proteintech), LS-C475-0.1 (LSBio), MAB8825 (Abnova), sc-12756, sc-74437, sc-74436, sc-377487, sc-69839, sc-69840, sc-53951 (all from Santa Cruz), and so on;
BCLw: MA5-15076 (Invitrogen), #2724 (CST), sc-293236, sc-130701 (all from Santa Cruz), NBP2-61706 (Novus), and so on;
BFL1: NBP2-67563, NBP2-46568CST (both from Novus), #14093 (CST), MA5-11757 (Invitrogen), sc-166943 (Santa Cruz), and so on;

### Pro-apoptotic proteins

BAX: ab32503, ab182733, ab81083 (all from Abcam), #5023, 2774S, 2772S (all from CST), MA5-14003, MA5-16322, 35-9700 (all from Invitrogen), BSM-52316R, BSM-33279M (both from Bioss), 50599-2-Ig (Proteintech), SAB4502546 (Merck), NB100-56096 (Novus), 610982, 610983 (both from BD), sc-7480, sc-23959, sc-70408 (all from Santa Cruz), CF810335, TA810334 (both from OriGene), #633602 (BioLegend), LS-C357719-20 (LSBio), and so on;
BAK: ab238515, ab32371, ab69404, ab137646 (all from Abcam), 6947S, 12105S (both from CST), B5897 (Merck), 06-536, AM03-100UGCN, SAB1305656 (all from Sigma), H00000578-M01A, MAB8753 (Abnova), sc-517390, sc-518110 (both from Santa Cruz), MA5-36225 (Invitrogen), GTX10808 (GeneTex), and so on;
BAD: 9296S (CST), sc-8044 (Santa Cruz), MA5-11117, MA5-15230 (both from Invitrogen), and so on;
NOXA: ab68523 (Abcam), sc-515840, sc-56169, sc-398873 (all from Santa Cruz), and so on; and
BIM: MA5-15763, MA5-15764 (both from Invitrogen), sc-374358 (Santa Cruz), CF812441 (OriGene), and so on.

The aforementioned antibodies are given only for illustration, but not limited thereto. So long as it is known to have binding activities to the specific proteins, any antibody may be used and expected to exhibit substantially the same effect.

The substance binding to the first protein-interacting protein or the first protein, used herein to detect the first protein-interacting protein or the first protein in the endogenous protein-protein interaction measuring step (step (ii)) may be labeled with a probe. When the binding substance is an antibody, the antibody or a secondary antibody to the antibody may be conjugated with a probe.

In addition to the step (a1) of measuring interaction between BCL2 family proteins and/or the step (a) of measuring a level of the BCL2 family proteins within the sample, the method provided herein may further comprise a step (b) of comparing a measurement of the interaction between BCL2 family proteins in step (a1) and/or a measurement of the level of the BCL2 family protein within the sample in step (a2) with a reference (b1), and/or identifying (determining, or deciding) a patient in which the sample or the sample-isolated subject is responsive to the BCL2 family protein-targeting drug and/or whether the BCL2 family protein-targeting drug exhibits medicinal efficacy in the sample or the sample-isolated subject, and/or selecting (identifying, determining, and deciding) the subject as a target suitable to be treated with the BCL2 family protein-targeting drug (b2).

The method provided herein may further comprise, subsequent to step (b) (more particularly step (b2)), a step of administering the BCL2 family protein-targeting drug to the subject which is identified in step (b) (more particularly step (b2)) to have response to the BCL2 family protein-targeting drug, to allow the drug to effect medicinal efficacy therein, or to be suitably treated with the drug.

As used herein, the term "responsiveness to the BCL2 family protein-targeting drug" or "medical efficiency of the drug" refers to a pathological effect that is obtained as the drug targets (e.g., inhibits) a target protein (i.e., BCL2 protein) and is intended to encompass anticancer effects (alleviation, reduction, relief, removal, and treatment of cancers (or cancer cells), and/or inhibition or delay of the progression and/or metastasis of cancer, etc.), in greater detail, anticancer effects on blood cancer.

The term "subject suitable to be administered the BCL2 family protein-targeting drug or for treatment with the drug", as used herein, refers to a patient in which the BCL2 family protein-targeting drug can exhibit a therapeutic effect on a disease. For example, the subject may be a patient in which the administration of the BCL2 family protein-targeting drug or the therapeutic treatment with the BCL2 family protein-targeting drug can exhibit anticancer effects (alleviation, reduction, relief, removal, and/or treatment of cancers or cancer cells).

The reference value in step (b) (in detail, step (b1)) may be established by the method provided herein, based on the information obtained from pre-existing cancer patients (e.g., blood cancer patients). In an embodiment, the reference value may be 1) a measurement obtained by applying the method (step (a1) and/or (a2)) provided herein to a patient or a group of patients in which treatment with the BCL2 family protein-targeting drug can exhibit therapeutic effects (patient or a group of patients responsive to the BCL2 family protein-targeting drug), or to a sample isolated from the patient or the group of patients (sample in an effective group), or 2) a measurement obtained by applying the method (step (a1) and/or (a2)) provided herein to a patient or a group of patients in which treatment with the BCL2 family protein-targeting drug cannot exhibit therapeutic effects (patient or a group of patients not responsive to the BCL2 family protein-targeting drug), or to a sample isolated from the patient or the group of patients (sample in a non-effective group).

In an embodiment where the reference value is obtained from a sample in an effective group, if the measurement obtained in the step of measuring interaction between BCL2 family proteins within a test sample (a1) and/or in the step of measuring a level of the BCL2 family proteins within a test sample (a2) is as high as or higher than the reference value (a value range around the reference value (e.g., the reference value itself or with an error range of 10% or less, 5% or less, 4% or less, 3% or less, 2% or less, or 1 % or less) or a value exceeding the reference value), the sample or the subject from which the sample is obtained may be selected as a patient responsive to the BCL2 family protein-targeting drug (or a patient who is suitable for treatment with the BCL2 family protein-targeting drug) or may be identified (determined, or decided) to be a patient in which the BCL2 family protein-targeting drug can exhibit medicinal effects (step (b2)).

In another embodiment where the reference value is obtained from a sample in a non-effective group, if the measurement obtained in the step of measuring interaction between BCL2 family proteins within a test sample (a1) and/or in the step of measuring a level of the BCL2 family proteins within a test sample (a2) is in a range around the reference value (e.g., the reference value itself or with an error range of 10% or less, 5% or less, 4% or less, 3% or less, 2% or less, or 1% or less) or less than the reference value, the same or the subject from which the sample is obtained may be selected as a patient who is not responsive to the BCL2 family protein-targeting drug (or a patient in which treatment with (administration of) BCL2 family protein-targeting drug does not exhibit medicinal effects) or may be identified (determined, or decided) to be a patient in which the BCL2 family protein-targeting drug cannot exhibit medicinal effects (step (b2)).

In another embodiment, the reference value may be established as a minimal value, a median value, or an average value of the measurements obtained in step (a1) of measuring interaction between BCL2 family proteins within the sample for an effective group and/or in step (a2) of measuring a level of the BCL2 family proteins within a sample for an effective group. The reference value may be adjusted with the addition of patient information or according to equations or algorithms derived from various analysis values. In step (b) of the method provided herein, the test sample or the subject from which the sample is obtained may be selected as a patient who is therapeutically responsive to the BCL2 family protein-targeting drug or who is suitable for treatment with BCL2 family protein-targeting drug when the measurement obtained in step (a1) and/or step (a2) is higher than (similar to and/or exceeding) the corresponding reference value.

In another embodiment, the reference value may be established as a maximal value, a median value, or an average value of the measurements obtained in step (a1) of measuring interaction between BCL2 family proteins within the sample for a non-effective group and/or in step (a2) of measuring a level of the BCL2 family proteins within a sample for a non-effective group. The reference value may be adjusted with the addition of patient information or according to equations or algorithms derived from various analysis values. In step (b) of the method provided herein, the test sample or the subject from which the sample is obtained may be selected as a patient in which the BCL2 family protein-targeting drug is not therapeutically effective when the measurement obtained in step (a1) and/or step (a2) is lower than (similar to and/or below) the corresponding reference value.

In another embodiment, the reference value may be a degree of interaction between a comparative protein within a sample and a protein interacting with the comparative protein and/or a level (expression level or concentration) of a comparative protein within a sample and/or a protein interacting with the comparative protein. The comparative protein and the protein interacting with the comparative protein may be selected from anti-apoptotic proteins and pro-apoptotic proteins. The comparative protein and the protein interacting with the comparative protein may be identical to or different from the first protein and the first protein-interacting protein described above, respectively. In an embodiment, the comparative protein may be different from the first protein while the protein interacting with the comparative example may be identical to or different from the first protein-interacting protein.

In this case, the method provided herein may further comprise the following step (a1') and/or (a2') prior to step (b):
(a1') measuring interaction between a comparative protein within a sample and a protein interacting with the comparative protein and/or (a2') measuring a level of a comparative example within a sample and/or a protein interacting with the comparative protein.

Provided according to another embodiment are a method for treatment of cancer (e.g., blood cancer), the method comprising a step of administering a BCL2 family protein-targeting drug to the following subject, and a pharmaceutical composition comprising a BCL2 family protein-targeting drug for cancer (e.g., blood cancer) therapy in the following subject through application (administration) thereof:
a subject in which the interaction between BCL2 family proteins within a sample obtained (isolated) from the subject (e.g., measured in (i) and/or (ii) in step (a1)), and/or a subject in which a level of a BCL2 family protein within the subject (e.g., measured in step (a2)) is similar to or exceeds the reference value (e.g., obtained from an effective group) (e.g., a subject which is identified to have responsiveness to the BCL2 family protein-targeting drug or to allow for the medicinal efficacy of the drug, as measured by the method, provided herein, for prediction of responsiveness to a BCL2 family protein-targeting drug and/or for prediction of medicinal efficacy of the drug, and/or a subject which is identified to be suitable for administration of a BCL2 family protein-targeting drug or for treatment with a BCL2 family protein-targeting drug as measured by the method for selecting a subject suitable for administration of a BCL2 family protein-targeting drug or for treatment with a BCL2 family protein-targeting drug).

BCL2 family proteins generally refer to proteins having BCL-2 homology domains (BH; BH1, BH2, BH3) in common, and function to regulate apoptosis (e.g., mitochondrial apoptosis).

As used herein, the first protein, which is a kind of BCL2 family protein, refers to an anti-apoptotic protein preventive of apoptosis, interacting with (binding to) an apoptotic protein and/or a pro-apoptotic protein to interfere with the function of the apoptotic protein and/or the pro-apoptotic protein. The first protein may be contained in a biological sample isolated from a subject or may be in a form separated from the sample. In an embodiment, the first protein may be a protein derived from mammals such as humans, etc. By way of example, the first protein may be at least one selected from the group consisting of BCL2 (e.g., NCBI ref seq. NM_000633.2, etc.), BCLxL (e.g., NCBI ref seq. NM_138578.1, etc.), MCL1 (e.g., NCBI ref seq. NM_021960.3, etc.), BCLw (e.g., NCBI ref seq. NM_001199839.2, etc.), and BFL1 (e.g., NCBI ref seq. NM_001114735.2, etc.).

The first protein-interacting protein may be at least one selected from the group consisting of: a BH3 subtype (BH3-only) protein (second protein); and a pro-apoptotic effector protein (third protein).

As used herein, the second protein (BH3 subtype protein) consists only of a BH3 domain and is thus called BH3-only protein. For example, the second protein may be at least one selected from the group consisting of BIM (e.g., NCBI ref seq. B033694), BID (e.g., NCBI ref seq. NM_001196.2), tBID (e.g., N-terminal truncated form of NCBI ref seq. NM_001196.2, etc.), BAD (e.g., NCBI ref seq. NM_004322.2, etc.), NOXA(e.g., NCBI ref seq. NM_001382616.1, etc.), PUMA (e.g., NCBI ref seq. AAO16862.1, etc.), BMF (e.g., NCBI ref seq. NM_001003940.2, etc.), BIK (e.g., NCBI ref seq. 1.NM_001197.5), and HRK (e.g., NCBI ref seq. 1.NM_003806.4, etc.).

As used herein, the third protein, which is a pro-apoptotic protein including multiple BH domains, may be at least one selected from the group consisting of, for example, BAX (e.g., NCBI ref seq. NM_138761.3, etc.), BAK (e.g., NCBI ref seq. NP_001179.1, etc.), and BOK (e.g., NCBI ref seq. NM_032515.5, etc.).

In another embodiment, the first protein may be at least one selected from the group consisting of a BH3 subtype (BH3-only) protein (second protein); and a pro-apoptotic effector protein (third protein), as stated above while the first protein-interacting protein may be at least one selected from anti-apoptotic proteins as stated above.

Herein, the externally provided first protein-interacting protein may be a wild-type protein or may be a variant protein having a sequence identity or homology of 50% or higher, 60% or higher, 70% or higher, 80% or higher, 90% or higher, 91% or higher, 92% or higher, 93% or higher, 94% or higher, 95% or higher, 96% or higher, 97% or higher, 98% or higher, or 99% or higher with the wild-type protein, with a proviso of being capable of interacting with the first protein. In addition, the first protein-interacting protein may be derived from humans or may be an xenogeneic protein capable of interacting with a human-derived first protein.

The subject may be a mammal, such as a human, etc. and, for example, may be a cancer patient (blood cancer, solid cancer) and more particularly, a blood cancer patient. For use in establishing a reference value, a subject may be required to be administered a BCL2 family protein-targeting drug or to be confirmed of medical efficacy of the drug therein.

Herein, blood cancer may be selected from the group consisting of leukemia (e.g., acute myeloid leukemia, acute lymphocytic leukemia, chronic myeloid leukemia, chronic lymphocytic leukemia, etc.), lymphoma (e.g., malignant lymphoma, B cell lymphoma, small lymphocytic lymphoma (SLL), etc.), multiple myeloma, and myelodysplastic syndrome.

The sample is a biological sample isolated (derived) from the patient and may include a cell, a cell lysate, a tissue, or a culture thereof, or an established cell line. For example, the cell or tissue may be a cancer cell or cancer tissue. In detail, the cancer may be blood cancer or solid cancer (e.g., breast cancer, lung cancer (e.g., adenocarcinoma of lung, etc.), and so on). In an embodiment, the subject may be a human patient with blood cancer while the sample may be at least one selected from the group consisting of leucocytes (e.g., granular leukocytes, lymphocytes, and/or monocytes) and myelocytes (hematopoietic stem cells, etc.), both isolated from patients with blood cancer or solid cancer (e.g., breast cancer or lung cancer (e.g., adenocarcinoma of lung, etc.), blood, bone marrow, and buffy coat which contain the cells, cancer cells, cancer tissues, and lysates thereof.

In one embodiment, the sample may be a lysate of at least one selected from the group consisting of leucocytes (e.g., granular leukocytes, lymphocytes, and/or monocytes) and myelocytes (hematopoietic stem cells, etc.), both isolated from patients with blood cancer or solid cancer (e.g., breast cancer or lung cancer (e.g., adenocarcinoma of lung, etc.), blood, bone marrow, and buffy coat which contain the cells, cancer cells, and cancer tissues. The lysate may be obtained using a solvent including at least one selected from the group consisting of HEPES, NaCl, glycerol, a cholesterol-based detergent (hereinafter, expressed as "surfactant"), and a phosphatase inhibitor cocktail. In this regard, the cholesterol-based detergent (e.g., glycol-diosgenin (GDN), digitonin, etc.), PEG-based detergent (e.g., Triton x-100, etc.), or the like may be employed. The detergent available in the present disclosure may include GDN, Digitonin), Triton x-100 (TX-100), or a combination thereof. For example, GDN may be used in an amount of 0.001-10 %, 0.005-5%, 0.008-1.2%, 0.01-10 %, 0.05-5%, 0.08-1.2%, 0.1-10 %, 0.5-5%, 0.8-1.2%, or about 1% (weight for weight), based on the total weight of the solvent. The lysate may be diluted into a proper concentration before use. The cholesterol-based detergent and/or the PEG-based detergent can stably maintain cellular functions and activity and/or three-dimensional protein structures in vitro in such a manner as in in vivo, compared to other types of detergents and allows proteins to retain relatively intact structures and/or functions as in cellular environments, making an advantageous observation of intracellular intrinsic protein characteristics even in in vitro.

In step (i), the externally provided (exogenous) first protein-interacting protein labeled with a probe may be used in a cell lysate form. The cell lysate may be obtained by treating a recombinant cell expressing the probe-labelled first protein-interacting protein with a suitable solvent, for example, a solvent including TX-100 or a cholesterol-based detergent. For example, TX-100 may be contained in an amount of 0.001%-1% or 0.2-5% (weight for weight), particularly 0.5-2%, more particularly 0.8-1.2%, and even more particularly 1%, based on the total weight of the solvent. The cell lysate thus obtained is diluted before application to a substrate. In this regard, the cell lysate may be adjusted to contain TX-100 in an amount of 0.02-0.04%. When containing TX-100 at a concentration greater than the upper limit, the solvent can reduce non-specific binding signals occurring at a PEG-coated substrate, but lower a measured value (signal value) observed from specific interaction between the first protein and the first protein-interacting protein, making protein analysis difficult. When TX-100 is used in an amount less than the lower limit, the dissolution of the first protein-interacting protein becomes unstable because TX-100 exists at less than a critical micelle concentration (CMC) in the solvent. As a result, non-specific binding signals generated by the first protein-interacting protein on the PEG-coated substrate increase, together with the measurement of ineffective signals that make protein analysis difficult. The critical micelle concentration is also true of the cholesterol-based detergent.

The concentration of the first protein-interacting protein in the cell lysate may range from 10⁻² to 10³ nM or from 10⁻¹ to 10² nM, but can be adjusted properly, without limitations thereto.

Herein, the BCL2 family protein-targeting drug may be at least one substance selected from the group consisting of proteins (e.g., antibodies, analogues thereof, etc.), nucleic acid molecules (e.g., siRNA, etc.), and small molecule compounds, each recognizing and/or inhibiting the BCL2 family proteins (e.g., anti-apoptotic proteins), and may be an apoptosis-controlling drug. In an embodiment, the BCL2 family protein-targeting drug may exhibit anticancer effects on, for example, blood cancer. Examples of BCL2 targeting drugs (e.g., BCL2 inhibitor) include an anti-BCL2 antibody, venetoclax, and ABT737. As a BCLxL targeting drug (e.g., BCLxL inhibitor), for example, an anti-BCLxL antibody, ABT263, or the like may be used. MCL-1-targeting drugs (e.g., MCL-1 inhibitors) may be exemplified by an anti-MCL-1 antibody, AMG-176, AZD5991, and Maritoclax.

The venetoclax (Venclexta^{®} or Venclyxto^{®}) can be used for treating acute myeloid leukemia (AML), chronic lymphocytic leukemia (CLL), small lymphocytic lymphoma (SLL), and so on.

Below, a detailed description will be given of the present disclosure.

An embodiment is explained with reference to venetoclax, which is representative of the BCL2 family protein-targeting drugs.

Proposed herein is a diagnostic method for blood cancer patients based on the quantification of the ability of BCL2 family proteins to inhibit apoptosis using single molecule protein interaction measuring method described above. Techniques essential therefor are as follows: (1) the observation of protein-protein interactions contemplates various states of BCL2 family proteins existing in cells, and (2) there is an ability to select cell lines and patients sensitive to BCL2 family protein-targeting drugs (e.g., venetoclax, AMG 176, or AZD5991) through multilateral observations of protein-protein interactions and quantitative comparison of protein-level observations.

### (1) Capturing BCL2 family protein onto substrate by maintaining intracellular state thereof

Herein, BCL2 family proteins endogenous to cells were captured onto a substrate while the intracellular states thereof are retained.

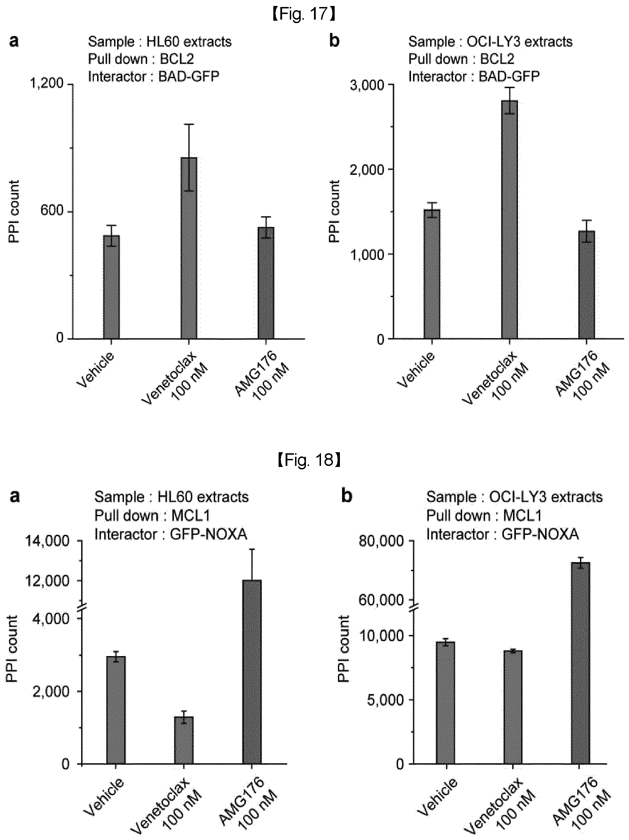

In an embodiment, a typical blood cancer cell line HL60 (acute myeloid leukemia) or the OCI-LY3 (B cell lymphoma) cell line were incubated with venetoclax to induce apoptosis. Then, a test was performed to measure the apoptotic protein BAX having the N-terminus revealed (exposed), which accounts for an activated conformation thereof. It was observed that the level of BAX having the N-terminus revealed increased with treatment with venetoclax. In contrast, after the N-terminus of BAX protein was artificially exposed by treating a cell lysate with TX-100, the detergent-activated BAX was observed to remain at a similar level, as measured by the same test. As is understood from the test, treatment with venetoclax increased the proportion of an activated conformation of BAX having the N-terminus exposed although not altering the expression level of BAX (see FIG. 17).

The result demonstrates that the method proposed herein can reflect the intact intracellular conformations/PTM (post-translational modifications) of BCL2 family proteins.

### (2) Identification that protein-protein interaction (PPI) accounts for state change of intracellular BCL2 family protein

Herein, it was confirmed that changes in the states of intracellular BCL2 family proteins are accounted for by PPI measurements.

In an embodiment, OCI-LY3 or HL60 cells were treated with the BCL2 inhibitor venetoclax or the MCL1 inhibitor AZD5991, after which changes in interaction between BCL2 family proteins (PPI) were compared. When applied to the cells, the inhibitors are anticipated to induce a state change (e.g., complex formation) in intracellular BCL2 family proteins because they selectively inhibit the interaction of BCL2 and MCL1 protein with other proteins. In practice, cell lysates were prepared from OCI-LY3 cells after incubation in the presence or absence of venetoclax (BCL2 inhibitor) or AZD5991 (MCL-1 inhibitor) for 24 hours. Then, BCL2 and MCL1 protein were extracted from the cell lines and analyzed for interaction with BAD and NOXA (both labeled with GFP). The BCL2-BAD interaction (PPI) for the venetoclax-treated cell lysate was measured to be higher than that for the cell lysate that were not treated with any inhibitors (non-treated group). In addition, a higher level was detected in interaction between MCL1 protein from the AZD5991 -treated cell lysate and NOXA (labeled)than between MCL1 protein from the non-treated group and NOXA (labeled);. By contrast, no influences were exerted to MCL1-NOXA interaction (PPI) by treatment with venetoclax and to BCL2-BAD interaction (PPI) by treatment with AZD5991. It was also proven that treatment with each drug does not incite a significant change in expression levels of BCL2 and MCL1 under the conditions used in this experiment.

That is, the identification that specific proteins undergo a state change in response to particular drugs and/or do not undergo a state change in response to proteins other than the particular drugs demonstrates that the method provided herein can analyze accurate state changes caused by drug treatment.

Conventional studies have been directed to the measurement of expression levels of BCL2 proteins to estimate the intracellular apoptotic activity of BCL2. However, the present disclosure is characterized by quantitative analysis for apoptosis inhibition through observation of the PPI including molecular traits of proteins.

### (3) Prediction of sensitivity to BCL2 family protein-targeting drug through observation of multilateral protein-protein interaction and protein level

With respect to the effect of pro-apoptotic proteins and anti-apoptotic proteins on apoptosis of cells or patient samples, quantitative measurements of interaction between proteins endogenous to the samples and foreign proteins (first protein-protein interaction), interaction between proteins, both endogenous to samples (second protein-protein interaction), and/or protein expression levels to analyze apoptosis signals in a multilateral aspect were performed. Using the analysis data, sensitivity to BCL2 family protein-targeting drug (e.g., venetoclax) was determined.

The measurement of interaction between an endogenous protein and an exogenous protein was based on the interaction between an anti-apoptotic protein, such as, BCL2, BCLxL, MCL1, etc., endogenous to a sample and a pro-apoptotic protein, such as BIM, BAD, NOXA, BAX, BAK, etc., provided from the outside (exogenous to the sample) (first protein-protein interaction, also referred to as "first PPI") and used for quantitative analysis for correlation with responsiveness to BCL2-targeting inhibitors (e.g., venetoclax) or MCL1-targeting inhibitors (e.g., AMG 176). For this, the externally provided pro-apoptotic protein was labeled with a probe.

The measurement of interaction between endogenous proteins (e.g., protein complex formation) was based on interaction between an anti-apoptotic protein, such as BCL2, BCLxL, MCL1, etc. and a pro-apoptotic protein, such as BAX, BAK, BIM, BAD, NOXA, etc., both endogenous to the same sample (second protein-protein interaction, also referred to as second PPI) and used for quantitative analysis for correlation with sensitivity to BCL2-targeting inhibitors (e.g., venetoclax) or MCL1-targeting inhibitors (e.g., AMG 176).

Comparative analysis of levels of interaction between endogenous proteins and exogenous proteins (first PPI) and/or interaction between endogenous proteins (second PPI) allowed the analysis of correlation with sensitivity to BCL2-targeting inhibitors (e.g., venetoclax) or MCL1-targeting inhibitors (e.g., AMG 176). Through the comparative analysis, states of anti-apoptotic proteins such as BCL2, BCLxL, MCL1, etc., endogenous to cells can be identified. For example, an exposure degree of an active site at which the anti-apoptotic protein interacts with a pro-apoptotic protein can be measured. From the measurement, binding degrees and binding affinity between proteins, remainder binding sites, drug sensitivity, etc. can be indirectly estimated.

Herein, levels of anti-apoptotic proteins, such as BCL2, BCLxL, MCL1, etc. or pro-apoptotic proteins, such as BAX, BAK, etc., both endogenous to the same cells, were measured to determine correlation with sensitivity to BCL2-targeting inhibitors (e.g., venetoclax) or MCL1-targeting inhibitors (e.g., AMG 176). The protein levels may be expression levels of the proteins, the activated proteins, or phosphorylated proteins.

The measurement of protein-protein interaction provided herein may be based on single-molecule pull-down and/or co-immunoprecipitation (co-IP).

As used herein, the term "protein-protein interaction" (PPI) refers to physical and/or chemical association between a first protein and a first protein-interacting protein or formation of a complex therebetween and may be quantitated in terms of at least one factor of, for example, binding frequency, binding intensity (affinity), and binding time. In addition, the term "interaction (binding) between a first protein and a first protein-interacting protein" is intended to encompass an interaction (binding) mediated by a different protein (positioned between the first protein and the first protein-interacting protein in a signaling pathway) as well as the direct interaction (binding) therebetween. Herein, the protein-protein interaction may be a single molecule reaction (reaction between one molecule of the first protein and one molecule of the first protein-interacting protein).

As described above, the first protein which is a BCL2 family protein (Anti-apoptotic protein) may be BCL-2 (e.g., NCBI ref seq. NM_000633.2, etc.), BCLxL (e.g., NCBI ref seq. NM_138578.1, etc.), or MCL-1 (e.g., NCBI ref seq. NM_021960.3, etc.). The first protein-interacting protein, which interacts with the first protein, may be at least one selected from the group consisting of BIM (e.g., NCBI ref seq. B033694), BID (e.g., NCBI ref seq. NM_001196.2) or tBID (e.g., NCBI ref seq. NM_001196.2 N-terminal truncated form, etc.), BAD (e.g., NCBI ref seq. NM_004322.2, etc.), BAX (e.g., NCBI ref seq. NM_138761.3, etc.), NOXA(e.g., NCBI ref seq. NM_001382616.1), and BAK(e.g., NCBI ref seq. NP_001179.1, etc.).

In the method provided herein, the step (a1; (i) and/or (ii)) of measuring an interaction between a first protein and a first protein-interacting protein may be performed ex vivo or in vitro for isolated cells or tissues.

Measurement by the present disclosure will be stepwise explained in detail.

### Step (1): Preparation of first protein-immobilized substrate (common to steps (i) and (ii))

In Step (1), a substrate having a first protein immobilized thereto is prepared by adding a first protein or a test sample containing the first protein to a substrate, the surface of which a substance capable of binding specifically to the first protein is anchored.

The sample may be a cell or tissue isolated from a subject (patient), a lysate, homogenate, or extract of the cell or tissue, or body fluid (e.g., blood (whole blood, plasma, or serum), saliva, etc.) from the subject. The patient may be a cancer patient, for example, a blood cancer patient. The sample may contain the cancer cells (or blood cancer cells). Concrete examples are as described above.

So long as it allows immobilization of the first protein to the surface of the substrate, any material and/or structure can be applied to the substrate (crystalline or non-crystalline material can be used). In an embodiment, the substrate may be made of a material that has a refractive index as high as or higher than the refractive index of water (about 1.3), which accounts for most parts of biological substances. In an embodiment, the substrate may range, in thickness, from about 0.1 to about 1 mm, from about 0.1 to about 0.5 mm, from 0.1 to about 0.25 mm, or from about 0.13 to about 0.21 mm, with a refractive index of about 1.3 to about 2, about 1.3 to about 1.8, about 1.3 to about 1.6, or about 1.5 to about 1.54. The substrate may be made of any material that meets the refractive index range. For example, the substrate may be made of a material selected from the group consisting of glass (refractive index: about 1.52) and quartz, but without limitations thereto. The substrate may be in any form that is typically used for observing a biospecimen and may be in a well-type form, a slide-type form, a channel-type form, an array form, a microfluidic chip form, or a microtube (capillary) form, but is not limited thereto. For microscopic observation, a cover glass may be mounted on a substrate to which the sample is applied. The material for the cover glass is as described above for the substrate, and may be as thin as or thinner than the substrate (for example, a refractive index of 1.52, a thickness of 0.17 mm, but without limitation thereto).

A substance binding specifically to the first protein may be any substance that can bind to the first protein, that is, BCL2, BCLxL, MCL1, or all of them. For example, the substance binding specifically to the first protein may be at least one selected from the group consisting of antibodies, antigen-binding fragments thereof (e.g., scFv, (scFv)2, scFv-Fc, Fab, Fab', F(ab')2, etc.), aptamers (proteins or nucleic acid molecules), small molecule compounds, each binding specifically to BCL2, BCLxL, MCL1, or all of them. In this regard, the substance binding specifically to the first protein (BCL2, BCLxL, MCL1, or all of them) may act on a site which does not interfere with the interaction between the first protein and the first protein-interacting protein, that is, a site other than the site at which the first protein interacts with (binds to) the first protein-interacting protein.

In an embodiment, the surface of the substrate may be suitably modified to include (anchor) a biological substance (e.g., antibody, etc.) binding specifically to the first protein thereon or the substrate may have a surface to which a substance binding specifically to the first protein is anchored. For surface modification, one side of the substrate may be treated (e.g., coated) with any compound having a functional group that can hold a biological substance (e.g., antibody, etc.) binding specifically to the first protein. For example, the substrate may be treated with a compound bearing a function group selected from the group consisting of an aldehyde group, a carboxyl group, and an amine group. In an embodiment, the compound bearing a functional group selected from the group consisting of an aldehyde group, a carboxyl group, and an amine group may be at least one selected from the group consisting of biotin, bovine serum albumin (BSA), biotinylated bovine serum albumin, polyethylene glycol (PEG), biotinylated PEG (PEG-biotin), and polysorbate (e.g., Tween20), but is not limited thereto. The surface-treated substrate may be further treated (e.g., coated) with at least one selected from the group consisting of neutravidin, streptavidin, and avidin.

### Step (2): reacting first protein with first protein-interacting protein

Step (2) may be carried by the following step (2-1) and/or step (2-2).

### Step (2-1): Interaction between protein endogenous to sample and protein exogenous to sample (first protein-protein interaction of step (i))

In step (2-1), a substrate (e.g., prepared in step (1)) having a first protein (endogenous protein) pulled down thereto is added with a probe-labeled (attached) first protein-interacting protein (exogenous protein) to allow a reaction therebetween. The signal generated from the probe can be measured to determine a level of the complex formed between the endogenous protein and the exogenous protein, which accounts for a level of interaction between the endogenous protein and the exogenous protein.

The labeled first protein-interacting protein means a form labeled with a probe that generates a detectable signal (chemically (e.g., covalently or non-covalently), recombinantly, or physically coupled with a probe) or conjugated with a tag to which a probe can bind. The detectable signal may be any signal (e.g., light, radioactive rays, etc.) that can be measured by detecting typical enzymatic reactions, fluorescence, luminance, and/or radiations. The probe may be any one selected from the group consisting of a small molecule compound, a protein, a peptide, and a nucleic acid molecule, which can each generate the signal. For example, the probe may be at least one selected from the group consisting of fluorescent dyes (small molecule compounds; Cyanine, Alex, Dylight, Fluoprobes, etc.), and fluorescent proteins (e.g., green fluorescent protein (GFP, enhanced GFP), yellow fluorescent protein (YFP), cyan fluorescent protein (CFP), blue fluorescent protein (BFP), red fluorescent protein (RFP), mcherry, etc.). So long as it is typically available in the art, any tag, such as His-tag/Ni-NTA, may be employed. In order to allow accurate and easy detection, without generation of noises, the concentration of the probe may be suitably determined within the range of up to about 1 µM, for example, 1 nM to 1000 nM, 1 nM to 500 nM, 1 nM to 100 nM, 10 nM to 1000 nM, 10 nM to 500 nM, or 10 nM to 100 nM, but without limitations thereto. The signals generated from the probes can be measured by any typically used detector (e.g., fluorescence microscope, fluorescence camera, fluorescence intensity measurement (quantification) device, etc.).

### Step (2-2): Interaction between endogenous proteins (second protein-protein interaction in step (ii))

In step (2-2), a substrate (e.g., prepared in step (1)) having a first protein pulled down thereto is added with a substance binding to a first protein-interacting protein (the binding substance labeled with a probe) to react the binding substance with the first-protein-interacting protein of a complex formed between the first protein (endogenous) and the first protein-interacting protein (endogenous). The signal generated from the probe can be measured to determine a level of the complex within the sample, which accounts for a level of the endogenous protein-protein interaction.

The substance binding to the first protein-interacting protein means a probe that generates a detectable signal (chemically (e.g., covalently or non-covalently), recombinantly, or physically coupled to the first protein-interacting protein) or which can bind to a tag. The detectable signal may be any signal (e.g., light, radioactive rays, etc.) that can be measured by detecting typical enzymatic reactions, fluorescence, luminance, and/or radiations. The probe may be any one selected from the group consisting of a small molecule compound, a protein, a peptide, and a nucleic acid molecule, which can each generate the signal. For example, the probe may be at least one selected from the group consisting of fluorescent dyes (small molecule compounds; Cyanine, Alex, Dylight, Fluoprobes, etc.), and fluorescent proteins (e.g., green fluorescent protein (GFP, enhanced GFP), yellow fluorescent protein (YFP), cyan fluorescent protein (CFP), blue fluorescent protein (BFP), red fluorescent protein (RFP), mcherry, etc.). So long as it is typically available in the art, any tag, such as His-tag/Ni-NTA, may be employed. In order to allow accurate and easy detection, without generation of noises, the concentration of the probe may be suitably determined within the range of up to about 1 µM, for example, 1 nM to 1000 nM, 1 nM to 500 nM, 1 nM to 100 nM, 10 nM to 1000 nM, 10 nM to 500 nM, or 10 nM to 100 nM, but without limitations thereto. The signals generated from the probes can be measured by any typically used detector (e.g., fluorescence microscope, fluorescence camera, fluorescence intensity measurement (quantification) device, etc.).

In order to more accurately measure protein-protein interaction, the method may further comprise a step of washing the substrate on which a reaction has been performed between the reaction step (step (2)) and the subsequent protein-protein interaction measuring step (step (3)).

### Step (3): protein-protein interaction measurement

In step (3), a signal is measured from the reaction mixture in step (2) (i.e., (2-1) and/or (2-2)). The signal measurement may be performed using any means that can detect (measure or identify) the signal (e.g., typically detectable signal such as enzymatic reactions, fluorescence, luminance, or radiation) generated by the probe used in step (2).

In an embodiment, the measurement of protein-protein interaction in step (3) may be based on real-time analysis.

In an embodiment, when the signal is a fluorescent signal, the signal detection may be achieved by using a fluorescence microscope, a fluorescence (measuring) camera, and/or a fluorescence intensity measurement (quantitation) device to image and/or quantitate the fluorescent signal generated by the probe to which absorbable light is provided.

When the signal is a fluorescent signal, step (3) (protein-protein interaction measuring step) may comprise the sub-steps of:
(i) providing a beam from a light source to the reaction mixture of step (2); and
(ii) detecting a fluorescent signal generated in response to the provision of the light beam from the light source.

In the sub-step (i) of providing a light beam from a light source, a light beam from a light source is provided to the reaction mixture of the first protein and the first protein-interacting protein in step (2). So long as this goal is achieved, no limitations are imparted to the time of providing a light beam from a light source. For example, the light beam may be continuously provided for a duration from a time prior to, concurrent with, or subsequent to step (1) until after step (2) or may be provided for a predetermined period of time just before, concurrently with, or just after step (2), but without limitations thereto.

So long as it emits a wavelength corresponding to the fluorescent signal, any light source may be used and may be exemplified by a laser, a halogen lamp, etc.

In an embodiment, step (3) of measuring a protein-protein interaction may be conducted by using a total internal reflection fluorescence (TIRF) microscope or a confocal microscope to provide a light beam and observe a fluorescent signal. In another embodiment, the total internal reflection fluorescence microscope may be equipped with a fluorescence (measuring) camera for signal imaging, for example, EMCCD (electron-multiplying charge-coupled device) or CMOS (complementary metal oxide semiconductor) and may be used to provide a light beam and image and/or quantitate fluorescent signals.

Step (3) of measuring a protein-protein interaction will be explained in more detail with reference to an example using a total internal reflection fluorescence microscope and a fluorescence camera, as follows:
A) the substrate of step (1) or (2) is mounted on the TRIF microscope. In a TRIF microscope, a light source is typically directed toward a substrate to be observed while the lens is positioned irrespective of the position of the light source;
B) the light source may be a laser, ranging in intensity from about 0.5 mW to about 5 mW, from about 0.5 mW to about 4.5 mW, from about 0.5 mW to about 4 mW, from about 0.5 mW to about 3.5 mW, from about 0.5 mW to about 3 mW, from about 0.5 mW to about 2.5 mW, from about 0.5 mW to about 2 mW, from about 1 mW to about 5 mW, from about 1 mW to about 4.5 mW, from about 1 mW to about 4 mW, from about 1 mW to about 3.5 mW, from about 1 mW to about 3 mW, from about 1 mW to about 2.5 mW, from about 1 mW to about 2 mW, from about 1.5 mW to about 5 mW, from about 1.5 mW to about 4.5 mW, from about 1.5 mW to about 4 mW, from about 1.5 mW to about 3.5 mW, from about 1.5 mW to about 3 mW, from about 1.5 mW to about 2.5 mW, or from about 1.5 mW to about 2 mW, and may have an intensity of about 2 mW. As stated beforehand, the light wavelength of the light source may be suitably selected according to fluorescent signals, probes used, and/or the configuration of the equipment (for example, an attenuation filter allows the use of a high-intensity light source).
C) fluorescent signals that are generated with the provision of light beams from the light source may be captured, imaged, and/or quantitated by means of a fluorescence (measuring) camera.

In consideration of the retention time of fluorescent signal generation (light-emission time, lifetime) of the probe, the capture (or imaging) of the fluorescent signals may be performed simultaneously with the provision of the light source or within the retention time of signal generation.

For capturing (or imaging) fluorescent signals with a fluorescent camera (e.g., EMCCD camera), exposure time, laser power, a camera gain value, total imaging frames, etc. may be suitably controlled. For example, a shorter exposure time per frame accumulates the signals on one frame to a lesser extent. To offset this, the power of the laser or the sensitivity of the fluorescent camera may be increased. In one embodiment, the exposure time per frame may be set to be about 0.001 sec to about 5 sec, about 0.001 sec to about 3 sec, about 0.001 sec to about 2 sec, about 0.001 sec to about 1 sec, about 0.001 sec to about 0.5 sec, about 0.001 sec to about 0.3 sec, about 0.001 sec to about 0.1 sec, about 0.01 sec to about 5 sec, about 0.01 sec to about 3 sec, about 0.01 sec to about 2 sec, about 0.01 sec to about 1 sec, about 0.01 sec to about 0.5 sec, about 0.01 sec to about 0.3 sec, about 0.01 sec to about 0.1 sec, about 0.05 sec to about 5 sec, about 0.05 sec to about 3 sec, about 0.05 sec to about 2 sec, about 0.05 sec to about 1 sec, about 0.05 sec to about 0.5 sec, about 0.05 sec to about 0.3 sec, about 0.05 sec to about 0.1 sec, about 0.07 sec to about 5 sec, about 0. 07 sec to about 3 sec, about 0. 07 sec to about 2 sec, about 0. 07 sec to about 1 sec, about 0. 07 sec to about 0.5 sec, about 0. 07 sec to about 0.3 sec, about 0.07 sec to about 0.1 sec, about 0.1 sec to about 5 sec, about 0.1 sec to about 3 sec, about 0.1 sec to about 2 sec, about 0.1 sec to about 1 sec, about 0.1 sec to about 0.5 sec, or about 0.1 sec to about 0.3 sec, for example about 0.1 sec, but is not limited thereto.

For example, when an EMCCD camera is used, photons generated from the probe (e.g., eGFP) are measured in the form of electrons converted through the element of the EMCCD (photoelectric effect). The number of electrons generated per photon may be changed through a gain value. The higher the gain value is set, the greater the number of electrons that are generated per photon, which results in increasing the sensitivity of EMCCD, with the concomitant increase of background noise. Hence, a signal-to-noise ratio may be important. In an embodiment, the gain value may be set to fall within a scope of, but is not limited to, about 10 to about 100, about 10 to about 80, about 10 to about 60, about 10 to about 50, about 20 to about 100, about 20 to about 80, about 20 to about 60, about 20 to about 50, about 30 to about 100, about 30 to about 80, about 30 to about 60, or about 30 to about 50, for example about 40, in order to obtain an excellent signal-to-noise ratio. However, the gain value may be suitably selected in consideration of the camera sensitivity and lifetime, equipment configuration conditions, noise test conditions, etc.

Multiplication of a total number of image frames by exposure time gives a total time of photographing (exposure time x total number of frames = photographing time). Because a fluorescent signal disappears after the emission time (lifetime) of a fluorescent material, the total number of image frames and/or the exposure time may be adjusted so that the photographing is conducted within the emission time (lifetime).

In order to increase the accuracy of protein-protein interaction measurements, the imaging is conducted on one or more, for example two or more, three or more, four or more, five or more, seven or more, or ten or more, substrates (the upper limit may be determined depending on the size of the substrate and an area in which imaging is allowed) or in one or more channels (each channel includes two or more substrates). The obtained fluorescent signals can be quantitated by measuring the number of signal-expressing spots (also referred to as PPI complex), which is regarded as a way to quantitate the protein-protein interaction.

In another embodiment, the quantitation of the protein-protein interaction may be achieved by digitizing the fluorescence intensity measured in step (3) by means of a typical device. For accuracy of data, the measurements may be normalized with reference to the sample amount or the protein amount in the sample.

When two or more different first proteins and/or first protein-interacting proteins are used, the steps (1) to (3) may be performed for each of the combinations of individual first and first protein-interacting proteins (that is, steps (1) to (3) may be performed repeatedly a number of times corresponding to the number of combinations of the first proteins and the first protein-interacting proteins).

The method provided herein may further comprise the following step (4).

### Step (4): Comparing step (step (b), more concretely (b1))

In step (4), the result (the level of protein-protein interaction) obtained in step (3) is compared with the level of interaction between the first comparative protein and the first comparative protein-interacting protein or with a reference value. The first comparative protein, the first comparative protein-interacting protein, and the reference value are as described above.

As used herein, the expression "targeting a BCL2 family protein" means recognizing (binding specifically to) a BCL2 family protein and/or inhibiting activity of a BCL2 family protein. Inhibiting activity of a BCL2 family protein accounts for reducing or eliminating the BCL2 family protein's intrinsic functions such as anti-apoptotic function by binding to the BCL2 family protein and/or by degrading and/or structurally modifying the BCL2 family protein.

The term "drug", as used herein, means any substance that exhibits a pharmacological effect, for example at least one selected from the group consisting of small-molecule compounds, proteins (e.g., antibodies, antibody fragments, analogs thereof, etc.), peptides, nucleic acid molecules (e.g., DNA, RNA (i.e., siRNA, microRNA, shRNA, etc.), PNA (peptide nucleic acid), aptamers, etc.), plant extracts, animal extracts, and cell extracts.

Herein, all of the externally provided proteins, such as a first protein-interacting protein, a fluorescent protein as a probe, an antibody as a binding substance, etc., may be produced by recombinant or chemical synthesis, but without limitations thereto. In an embodiment, the recombinant production may include a step of transfecting a gene coding for a protein of interest or a vector carrying the gene into mammalian cells such as HEK293 cells, insect cells such as SF9, or bacterial cells such as E. coli and then allowing the cells to express the protein. The expressed protein may undergo a further purification process which, however, may not be essential. The purified protein or the cell lysate containing the expressed protein may be used, as it is, to conduct the measurement of protein-protein interaction.

As used herein, the terms "BCL2 family protein-targeting therapy" and "therapy with a BCL2 family protein-targeting drug" can be interchangeably used with each other, with an equivalent meaning therebetween, and are intended to encompass all medical and/or pharmaceutical behaviors leading to inhibiting the activity of the first protein. For example, the therapy may include prescription and/or administration of a drug inhibitory of the activity of a BCL2 family protein to a subject in need thereof. In greater detail, "BCL2 family protein-targeting therapy" may include prescription and/or administration of a drug that binds to, degrades, and/or structurally modifies a BCL2 family protein to reduce or eliminate intrinsic functions, for example, an anti-apoptotic function to a subject in need thereof.

The administration may be carried out via an oral or parenteral route. The parenteral administration may be performed by intravenous injection, subcutaneous injection, intramuscular injection, intraperitoneal injection, intradermal administration, intraregional topical administration, intranasal administration, intrapulmonary administration, or intrarectal administration.

Examples of the individual, patient, or subject may include all mammals, for example, primates such as humans, monkeys, etc., rodents such as mice, rats, etc., and the like, and may be a patient with a first protein-related disease. The first-protein-related disease may be a disease associated with the overexpression of a first protein or with the activation of a signaling pathway in which a first protein is involved in a cell or tissue, for example, cancer and more particularly blood cancer. In an embodiment, the individual, patient, or subject may be a cancer patient and particularly, a blood cancer patient.

As used herein, the term "responsiveness to a drug" means the degree of the effect that the drug exhibits in a drug-administered individual.

As used herein, the term "effect" or "efficacy" refers to a medical and/or pharmaceutical effect that a drug or therapy is intended to achieve in a treated subject and may mean prevention and/or treatment of a disease in a subject and/or reduction and/or alleviation of a disease symptom. For example, when the drug, the treatment, and the subject are an anticancer agent, an anticancer therapy, and a cancer patient, respectively, the effect may be an anticancer effect (preventive and/or therapeutic effect on cancer). The anticancer effect may include effects of preventing migration, invasion, and/or metastasis of cancer, suppressing deterioration of cancer, and/or reducing or eliminating resistance of cancer as well as inhibiting the growth of cancer cells.

In another embodiment, an analysis device is provided for predicting an effect of a BCL2 family protein-targeting drug and/or for use in a therapy with a BCL2 family protein-targeting drug.

The analysis device may comprise:
a reaction part including therein a first protein or a substance for capturing a first protein by binding specifically to the first protein; and
optionally an agent for detecting a first protein-interacting protein and/or a signal detection means. The agent for detecting a first protein-interacting protein contains a substance (specifically) binding to the first protein-interacting protein. The binding substance is a binder (e.g., antibodies, antigen-binding fragments, antibody analogs, aptamers, small molecule compounds, so on) which binds to a site different from that at which the first protein-interacting protein interacts with (binds to) the first protein, and may be labeled with a probe.

The first protein is as described above and may be used as a purified protein or included in cells or a cell lysate. The reaction part may comprise the first protein, cells or a cell lysate, each including the same, or a substrate having the same immobilized thereto.

The analysis device may be adapted to determine interaction between the first protein and the first protein-interacting protein qualitatively and/or quantitatively.

The reaction part may comprise a substrate to which a substance for capturing the first protein is immobilized or to which the first protein is immobilized directly or via the capturing substance. The substance for capturing a first protein may be a substance, for example, an antibody or an antigen binding fragment, which binds specifically to the first protein. The reaction part may be in a well-type (e.g., multi-well-type) form, a slide-type form, a channel-type form, an array form, a microfluidic chip form, or a microtube (capillary) form, but without limitations thereto.

The analysis device may be adapted to measure an interaction between the first protein and the first protein-interacting protein. In this context, the analysis device may further comprise the first protein-interacting protein. In an embodiment, the first protein-interacting protein may be labeled with a probe that generates a detectable signal (coupled with a probe, for example, chemically (e.g., covalently or non-covalently), recombinantly, or physically) or may have a tag attached thereto, the tag being capable of being coupled to a probe.

In addition, the protein-protein interaction measuring device may further comprise a probe binding to the first protein and a labeling substance capable of being coupled to the probe in order to normalize a signal value measured by the signal detection means to the level of the first protein. The probe binding to the first protein may be a biomolecule (e.g., antibody) that binds to the first protein at a site different from that for the capturing substance contained in the aforementioned multi-wells. The labeling substance may be coupled to a probe that generates a detectable signal (coupled to a probe, for example, chemically (e.g., covalently or non-covalently)) and may be a biomolecule (e.g., antibody) capable of being coupled to the probe binding to the first protein.

The signal detection means may be any signal detection means that is typically used according to the signals generated by the probe used. For example, the signal detection means may comprise a signal stimulation part and a signal detection part. Further, a signal analysis part for analyzing (e.g., quantitating or imaging) measured signals may be included. In one embodiment, signal stimulation, signal detection, and signal analysis may be conducted at respective regions. Alternatively, at least two of signal stimulation, detection, and analysis may be conducted simultaneously or consecutively in one region. In one embodiment, when the probe is a fluorescent material, the signal detection means may be selected from all means that can generate and detect a fluorescent signal, and may comprise, for example, a fluorescent signal stimulation part (e.g., light source) and a fluorescent signal detection part, and/or a fluorescent signal analysis part. In one embodiment, the signal detection means may comprise a Total Internal Reflection Fluorescence (TIRF) microscope or a confocal microscope (light source and fluorescent signal detection) and optionally a fluorescence camera, for example, an electron-multiplying charge-coupled device (EMCCD) camera or a complementary metal oxide semiconductor (CMOS) camera to provide a light source and image and/or quantitate fluorescent signals. The wavelength and strength of the light source and the measurement conditions of a fluorescence camera (e.g., exposure time per frame, laser power, camera gain value, total frame numbers, etc.) are as described above.

The protein-protein interaction measuring device provided herein has the advantage of being able to accurately and effectively observe, analyze, detect, and/or measure interaction between a first protein and a first protein-interacting protein and/or expression levels of a first protein and a first protein-interacting protein even using a small amount of a sample. Therefore, the multi-well and the analysis method using the same can be usefully and effectively applied even to a very small amount of a biopsy (e.g., needle biopsy) sample.

### [EFFECT OF THE INVENTION]

BCL2 family protein-targeting drugs, such as venetoclax, etc., can be used for treating various blood cancer patients. For example, venetoclax can be applied to the treatment of acute leukemia and bone marrow cancer. However, there is a need for a diagnostic technique for predicting efficacies of the drugs in order to alleviate the burden laid on the patients. Conventional methods of determining expression levels of single BCL2 proteins and the presence or absence of mutant genes cannot succeed in predicting efficacies of BCL2 family protein-targeting drugs, such as venetoclax, etc. In contrast, the protein-protein interaction- and/or protein expression-based method provided herein can successfully predict in a fast and specific manner whether or not BCL2 family protein-targeting drugs, such as venetoclax, etc., effectively work on individual patients with various blood cancers, thereby allowing for quick and effective tailored personal therapy for blood cancers.

### [BRIEF DESCRIPTION OF THE DRAWINGS]

FIG. 1 shows measurements of interaction between individual antibody immobilized to a substrate and target proteins. FIG. 1a relates to BCL2, BCLxL and MCL1, and FIG. 1b relates to BCL-W and Bfl-1.
FIG. 2a shows PPI counts between endogenous proteins BCL2, MCL1 and BCLxL and exogenous proteins BIM, NOXA, and BAD. FIG. 2b shows PPI counts between endogenous proteins BCL2 and BCL-XL and exogenous protein tBID. FIG. 2c shows PPI counts between endogenous proteins BCL2, MCL1, and BCL-XL and exogenous proteins BAX and BAK, and FIG. 2d shows PPI counts between endogenous BCL2, BCL-XL, and MCL1 and only the BH3 domain of exogenous BIM protein as a probe.
FIG. 3 shows changes in PPI counts depending on the concentrations of the first protein-interacting protein by measuring the interaction between endogenous BCL2 and exogenous BAD.
FIG. 4 shows changes in PPI count according to types and concentrations of detergents contained in solutions provided from the outside by measuring the interaction between endogenous BCL2 and exogenous BAD.
FIG. 5 shows changes in PPI counts according to concentrations of the detergent Triton X-100 contained in solution provided from the outside by measuring the interaction between endogenous BCL2 and exogenous BAD.
FIG. 6 shows that when mutations occur at the highly conserved amino acids among species with respect to the exogenous BIM protein, PPI counts between the BIM variants and BCL-XL or BCL2 differ from one variant to another.
FIG. 7 shows complex counts between BCL2, MCL1, or BCL-XL and BIM, all endogenous to the samples (HL60 and OCI-LY3).
FIG. 8 shows complex counts between BCL2 and BAX, both endogenous to the sample (OCI-LY3).
FIG. 9 shows complex counts between BCL2, BCL-XL, or MCL1 and BAK, all endogenous to the sample (OCI-LY3).
FIG. 10 shows complex counts between BCL-XL and BAD and between MCL1 and NOXA, all endogenous to the samples (HL60, OCI-LY3, and SUDHL8).
FIG. 11 shows that the first protein and the first protein-interacting protein are interchangeable with each other for measuring protein complex counts.
FIG. 12a shows that both of two different types of BAK antibody can bind to BAK. FIG. 12b shows signal measurements obtained using the indicated pull-down antibody (Ab) for cells (HEK293) in which [MCL1, BAK], [BCL-XL, BAK], or [BCL2, BAK] are co-expressed, thereby demonstrating that the BAK antibody of D4E4 clone, which was capable of binding to BAK, can no longer bind to the BCL2 family proteins when co-expressed. FIG. 12c shows that the co-expressed BAK proteins can be detected using AT38E2, which is one of the antibodies used in FIG. 12a.

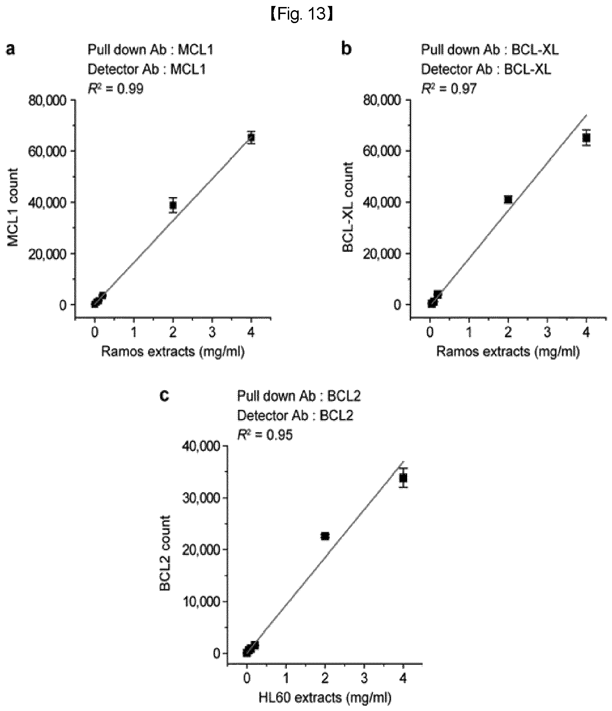

FIG. 13 shows expression levels of MCL1, BCL-XL, and BCL2, all endogenous to the sample.
FIG. 14 shows expression levels of BAX and BAK, both endogenous to the sample.
FIG. 15 shows BAX expression levels measured using a detection antibody after addition of therapeutic agents (Staurosporine and Venetoclax) in a GDN-treated sample (a), a Triton X-100-treated sample (b), a GDN-treated sample (c), and a Triton X-100-treated sample (b).
FIG. 16 shows expression levels of BAX of specific folded conformations in a specimen with respect to the treatment time with venetoclax (a and c) and treatment time with Triton X-100 (b and d).
FIG. 17 shows PPI counts between BCL2 endogenous to a sample (HL60) and exogenous BAD after treatment with venetoclax or AMG176 (a) and between BCL2 endogenous to a sample (OCI-LY3) and exogenous BAD after treatment with venetoclax or AMG176.
FIG. 18 shows PPI counts between MCL1 endogenous to a sample (HL60) and exogenous NOXA after treatment with venetoclax and AMG176 (a) and between MCL1 endogenous to a sample (OCI-LY3) and exogenous NOXA after treatment with venetoclax or AMG176.
FIG. 19 shows PPI counts between BCL-XL endogenous to a sample (HL60) and exogenous BAD after treatment with venetoclax and AMG176 (a) and between BCL-XL endogenous to a sample (OCI-LY3) and exogenous BAD after treatment with venetoclax or AMG176.
FIG. 20 shows expression levels of BCL2, MCL1, and BCL-XL in a sample (HL60) after treatment with venetoclax and AMG176 (a) and in a sample (OCI-LY3) after treatment with venetoclax or AMG176 (b).
FIG. 21 shows complex counts between BCL2, MCL1, or BCL-XL and BIM, all endogenous to a sample (HL60) after treatment with venetoclax or AMG176 (a) and between BCL2, MCL1, or BCL-XL and BIM, all endogenous to a sample (OCI-LY3) after treatment with venetoclax or AMG176.
FIG. 22 shows complex counts between MCL1 and NOXA, both endogenous to a sample (HL60) after treatment with venetoclax or AMG176 (a) and between MCL1 and NOXA, both endogenous to a sample (OCI-LY3) after treatment with venetoclax or AMG176.
FIG. 23 shows complex counts between BCL-XL and BAD, both endogenous to a sample (HL60) after treatment with venetoclax or AMG176 (a) and between BCL-XL and BAD, both endogenous to a sample (OCI-LY3) after treatment with venetoclax or AMG176.
FIG. 24 shows complex counts between BCL2 and BAX, both endogenous to a sample (HL60) (a) with respect to the treatment time with venetoclax and between BCL2 and BAX, both endogenous to a sample (OCI-LY3) with respect to the treatment time with venetoclax.
FIG. 25 shows BAX expression levels with respect to the treatment time with venetoclax in a sample (HL60) (a) and in a sample (OCI-LY3) (b).
FIG. 26 shows BAK expression levels with respect to the treatment time with venetoclax in a sample (HL60) (a) and in a sample (OCI-LY3) (b).
FIG. 27 shows PPI counts between BCL2 endogenous to a sample (HL60) and exogenous BIM according to the dosage of venetoclax or AZD5991.
FIG. 28 shows PPI counts between MCL1 endogenous to a sample (HL60) and exogenous BIM according to the dosage of venetoclax or AZD5991.
FIG. 29 is a Z-score heatmap showing measurements of the parameters (interaction (first PPI), complex (second interaction), and expression level) in specimens from AML patients (n=32).
FIG. 30 shows quantitative measurements of BCL2 expression in specimens from breast cancer patients (n=302).
FIG. 31 shows quantitative measurements of BCL2 expression in specimens from lung carcinoma patients (n=15).
FIG. 32 shows PPI counts and complex counts between endogenous BCL2 and exogenous BIM with respect to the treatment time of the sample (OCI-LY3) with venetoclax.
FIG. 33 shows PPI counts and complex counts between endogenous MCL1 and exogenous NOXA with respect to the treatment time of the sample (OCI-LY3) with AZD5991.
FIG. 34 shows PPI counts between BCL2, MCL1, or BCL-XL, all endogenous to the samples from patient 1, and exogenous BIM, as measured in i) the bone marrow acquired at the time of diagnosis, ii) the bone marrow acquired again after treatment with a FLT3-targeting anticancer agent (quizartinib), and iii) a whole blood specimen acquired after treatment with venetoclax and LDAC.
FIG. 35 shows PPI counts between BCL2, MCL1, or BCL-XL, all endogenous to the samples from patient 2, and exogenous BIM, as measured in i) the bone marrow acquired at the time of diagnosis, ii) the bone marrow acquired again after treatment with a FLT3-targeting anticancer agent (quizartinib), and iii) a whole blood specimen acquired after treatment with venetoclax and LDAC.
FIGS. 36a to 36c shows the correlation analysis results of responsiveness to venetoclax (vertical axis) with 21 parameters including PPI (first PPI), complex (second PPI), expression level, etc. in specimens from AML patients (n=32).
FIG. 37 shows the correlation analysis results of responsiveness to venetoclax with parameters including BCL2-BIM PPI (first PPI) and BCL2-BIM complex (second PPI) in specimens from AML patients.
FIG. 38 shows prediction for responsiveness to venetoclax, based on combinations of parameters. FIG. 38a shows correlation coefficients as the number of parameters increases from 1 to 10. FIG. 38b shows correlation analysis results for a combination of two indices, compared to single indices. FIG. 38c shows correlation analysis results for a combination of five parameters.
FIGS. 39a to 39c show the correlation analysis results of responsiveness to AZD5991 with 21 parameters including PPI (first PPI), complex (second PPI), expression level, etc. in specimens from AML patients (n=26).
FIG. 40 shows prediction results for responsiveness to AZD5991 using a combination of four main indices.

### [EXAMPLES]

Hereafter, the present invention will be described in detail by examples.

The following examples are intended merely to illustrate the invention and are not construed to restrict the invention.

### EXAMPLE 1. Measurement of Protein-Protein Interaction (PPI) between Endogenous Protein and Exogenous Protein

### 1.1. Preparation of analysis lysate (sample)

From bone marrow samples obtained from patients with various blood cancers such as chronic lymphocytic leukemia (CLL) and acute myeloid leukemia, only buffy coats were selectively harvested using a Ficoll gradient separation protocol (GE healthcare Ficoll-Paque plus 17-1440-03).

Pellets of the separated buffy coats were washed with dPBS, followed by separation with 500 g of dPBS for 5 min. This procedure was repeated twice to remove as many erythrocytes and platelets remaining in buffy coats as possible. The blood cancer cells thus separated from buffy coats were aliquoted by 10⁷ cells and the aliquots were snap-frozen in liquid nitrogen and stored at -80°C until use in experiments.

Blood cancer cells (The Korean Cell Line Bank and ATCC; HL-60 (CVCL_0002), OCI-LY3 (CVCL_8800)), Ramos (CVCL_0597), U937, THP1, and SUDHL8) and the blood cancer cell pellets from buffy coats of patients were each suspended in a lysis buffer (HEPES 50mM NaCl 150mM Glycerol 10%(w/v) GDN(Digitonin) 1%(w/v) with 2%(w/v) phosphatase inhibitor cocktail 2/3 (P5726, P0044)) and vortexed three times at regular intervals of 10 min. After 30 min, the lysis buffer containing the pellet homogenate was centrifuged at 15,000 g for 10 min. The supernatant thus formed was used as an analysis lysate to be applied to a substrate in subsequent experiments.

### 1.2. Preparation of first protein-interacting protein labeled with probe

A probe-labeled first protein-interacting protein (probe protein) was produced through transient transfection. In brief, after digestion of pEGFP-C1 (Addgene: PT3028-5) with restriction enzymes, genes coding for proteins BIM (Sino biological: HG13819-G), NOXA (Sino biological: HG16548-U), and BAD (Sino biological: HG10020-M) were each inserted into the digested plasmid to construct GFP expression protein vectors. The expression vectors were transiently transfected into HEK293T cells (ATCC; CRL-3216) using polyethylenimine to afford overexpressed GFP-labeled proteins.

The HEK293T cells in which the GFP-labeled proteins were expressed were lysed in 1% TX100 lysis buffer. The cell lysates thus prepared were each diluted into a concentration of 165 nM in 1% TX100 lysis buffer. Aliquots of the dilution were snap frozen and thawed just before experiments. Finally, the cell lysate containing the labeled first protein-interacting protein was prepared to have TX-100 at a concentration of 0.03%.

Labeled first protein-interacting proteins of BID or the only BH3 domain of BIM, BAX, or BAK were prepared using the method of the present disclosure. The first protein-interacting proteins (human derived) used in the following Examples are given, together with their Uniprot IDs and amino acid regions, in Table 1, below.

**TABLE 1**

| First protein-interacting protein | Uniprot ID |
|---|---|
| BAD | Q92934 |
| NOXA | Q13794 |
| BIM | Q43521 |
| BID | P55957 |
| BIM_BH3 | Q43521 (AA 134-166) |
| BAX_BH3 | Q07812 (AA 59-73) |
| BAK_BH3 | Q16611 (AA 74-88) |

As a probe for the first protein-interacting protein, green fluorescent protein (hereinafter referred to as "GFP"; labeled using pEGFP-C1 vector (Clontech)) was used to link eGFP to the C-terminus of the first protein-interacting protein.

### 1.3. Preparation of substrate

A glass coverslip with a size of 45 mm × 60 mm was coated with a mixture of 3:100 (w:w) of biotin PEG: mPEG.

For use as a mediator to pull a BCL2 family protein (first protein) down to the PEG-coated surface of the substrate, NTV (Neutravidin; Thermo, A2666) and a biotinylated goat anti-rabbit secondary antibody (111-065-046 Jackson Immunoresearch) were employed.

After coupling NTV and the secondary antibody to the PEG-coated substrate, primary antibodies, listed in Table 2 below, for pull down corresponding BCL2 family proteins (first proteins) were applied to the substrate.

Lysates of patent cells and cell lines (Korean Cell Line Bank and ATCC; HL-60, U937, THP1, OCI-LY3, and SUDHL8) were each put in an amount of 10 µl to each well coated with the antibody (see Table 2) for pulling down a protein to be observed and incubated for one hour at room temperature, followed by washing off excess lysates with washing buffer (HEPES 50 mM NaCl 150 mM glycerol 1% GDN 0.03%).

**TABLE 2**

| First protein (Human) | Uniprot ID | Information on First protein-binding substance (Antibody) |
|---|---|---|
| BCL2 | P10415 | #4223 (CST) |
| BCL-XL | Q07817 | MA5-15142 (Thermo Fisher Scientific) |
| MCL1 | Q07820 | #94296 (CST) |
| BCLw | Q92843 | MA5-15076 (Invitrogen) |
| BFL1 | Q16548 | NBP2-67563 (Novus) |

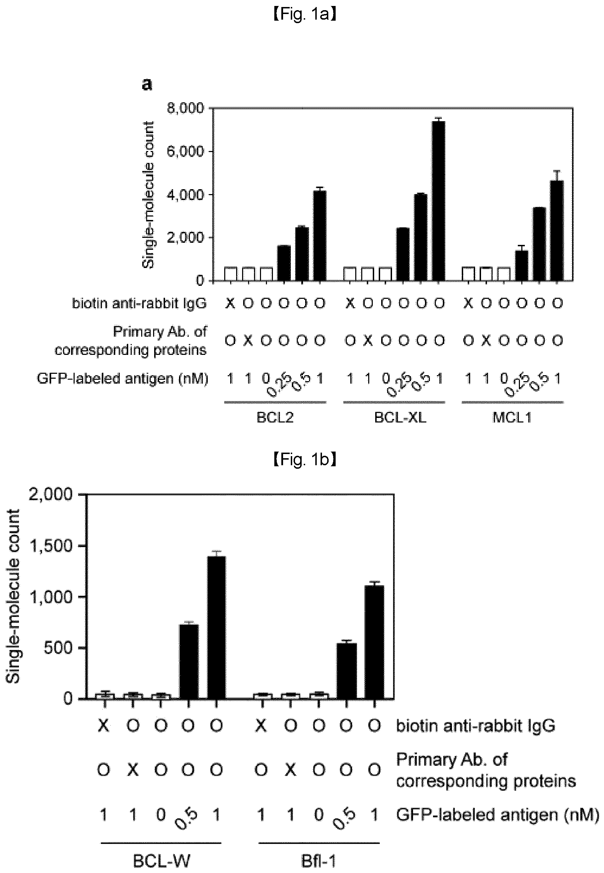

The performance of the antibodies given in Table 2 was measured in terms of the degree of pulling down GFP-fused first proteins onto the substrate. Briefly, 1 nM of a GFP-fused BCL2 protein was applied to the antibody-coupled substrate and incubated. After washing, remaining GFP was quantitatively measured. The same experiment was performed for BCL-XL, MCL1, BCLw, and BFL1, too. Results thus obtained are depicted in FIGS. 1a and 1b. As shown in FIGS. 1a and 1b, the antibodies given in Table 2 were observed to successfully pull down the respective target proteins onto the substrate.

### 1.4. Measurement of endogenous protein and exogenous protein

The cell lysate, prepared in Example 1.2, containing the probe-labeled first protein-interacting protein was added to the substrate prepared in Example 1.3. Briefly, 10 µl of the cell lysate of Example 1.2 was added to each well and incubated for 15 min at room temperature. Excess lysate was washed off with a washing buffer (HEPES 50mM NaCl 150mM glycerol 1% GDN 0.03%). Then, protein-protein interactions were measured as follows:
The substrate was fixedly mounted on a total internal reflection fluorescence and imaged to obtain data for protein-protein interaction (binding). To increase the accuracy of results, images were acquired from a total of 10 different positions under the same condition and an average thereof was used as a representative value.

Fluorescence images were stored in a 16-bit unsigned integer format. For use in observing the signals, a laser was given a suitable wavelength (488 nm for GFP). The laser power was adjusted to 2 mW to maintain the luminescence of eGFP for about 11 sec.

Of all the frames (10 frames), early and middle frames were discarded, and an average of the images of three frames (7^{th}-9^{th} frames) was taken to generate one image. This process was repeatedly conducted at various positions within a well to acquire a total of ten images with which the following procedures were then performed. In this Example, an EMCCD (electron-multiplying charge-coupled device; Andor iXon Ultra 897 EX2 (DU-897U-CS0-EXF)) camera was used to obtain fluorescence images with an exposure time of 0.1 per frame and an EMCCD gain value of 40.

The signals from the fluorescent protein were detected as a localized point spread function (PSF). A number of PSF (physical value) is a PPI score (biological value) between a BCL2 family protein and a protein to be measured. The PSF value was converted into a PPI score, which is a biological value, via the following procedures:
(a) Positions of local maxima were obtained (for example, i^{th} row, j^{th} column pixel). As described beforehand, single-molecule fluorescence signals were localized and formed at a particular position (about 5x5 pixel size, 1 pixel = 0.167 micrometers, under the current observation equipment). Hence, the discovery of local maxima allows the selection of individual PSF. This can be obtained using a toolkit provided by MATLAB.
(b) A process of determining whether or not the local maxima obtained in (a) were actually generated from PSF was performed. To begin with, a minimum intensity value of the local maxima was defined. Analysis was conducted only in the case where the local maxima obtained above were greater than the minimum intensity value. The minimum intensity value used in this Example was 75, and may vary depending on laser power/exposure time/established equipment state. Information of 5x5 pixels based on the finally obtained local maximum coordinates was retrieved and the centroid of intensity was determined in the 5x5 pixels. Here, if the obtained centroid of intensity deviated by 0.5 pixels or greater from the existing local maximum coordinate (if 2D symmetry for the PSF pattern disappeared), the fluorescent signal was determined to be abnormal and excluded from analysis.
(c) Only the PSF that passed all test conditions were selected for determination of the coordinates and the total number. Herein, the total number of PSF obtained becomes a PPI count.

Every file photographed under the same condition was subjected to the above procedure to obtain numbers of PSF which were then aggregated and used to calculate a mean and standard deviation. This value finally represents a PPI count in a particular condition. In this Example, complexes formed between exogenous first protein and exogenous first protein-interacting proteins could be estimated through the PPI counts.

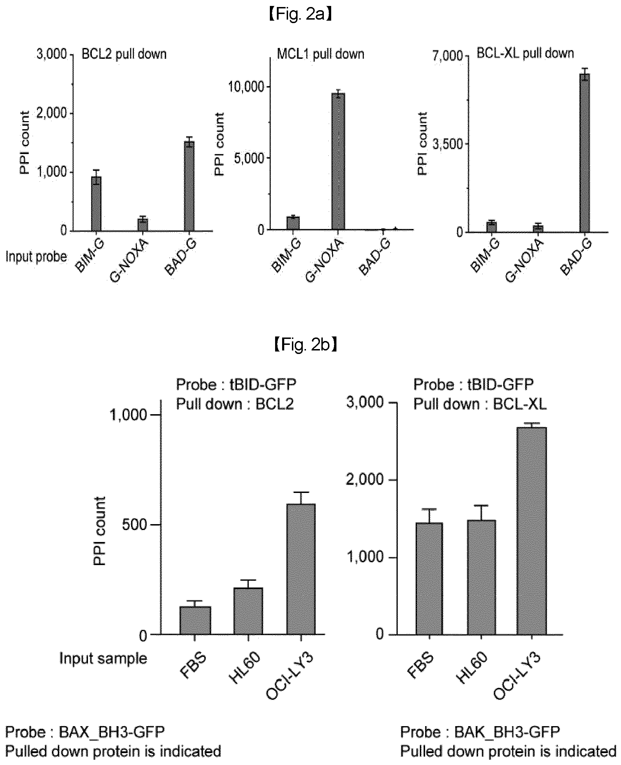

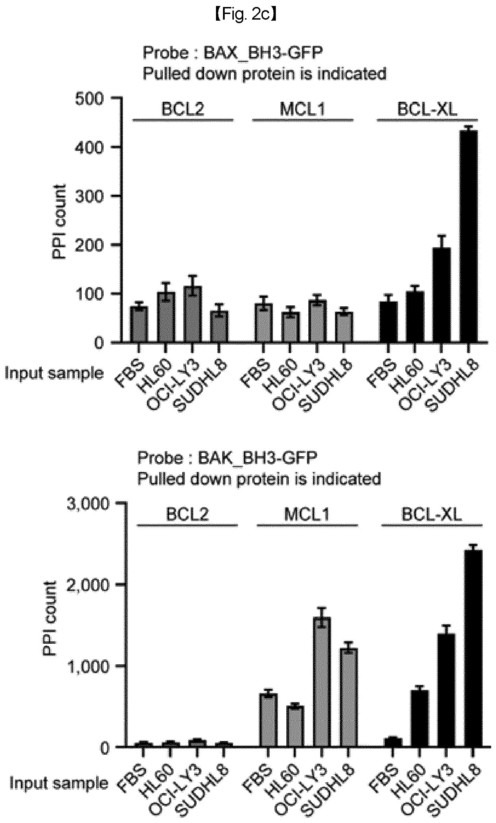

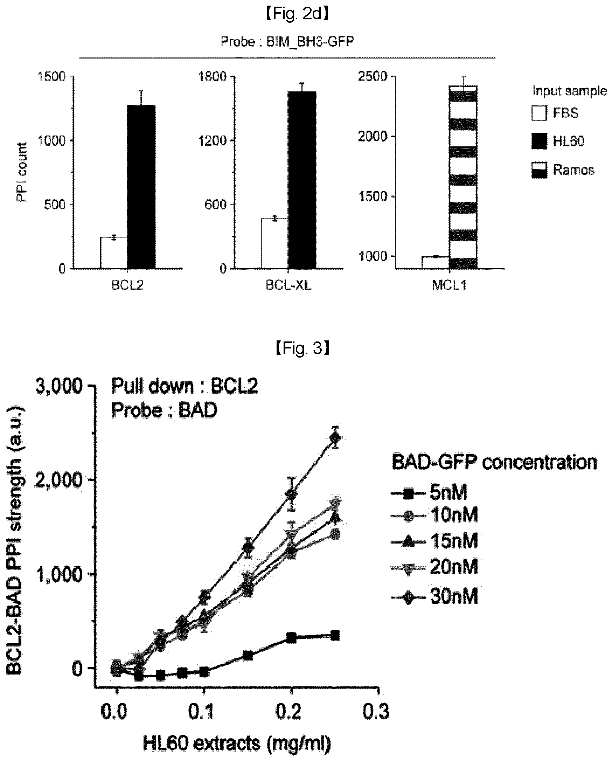

Measurements of the PPI counts are depicted in FIGS. 2a (results according to kinds of first proteins and first protein-interacting proteins), 2b (results according to kinds of cell lines), 2c (results from according to kinds of first proteins and cell lines), and 2d (results according to kinds of first proteins and cell lines).

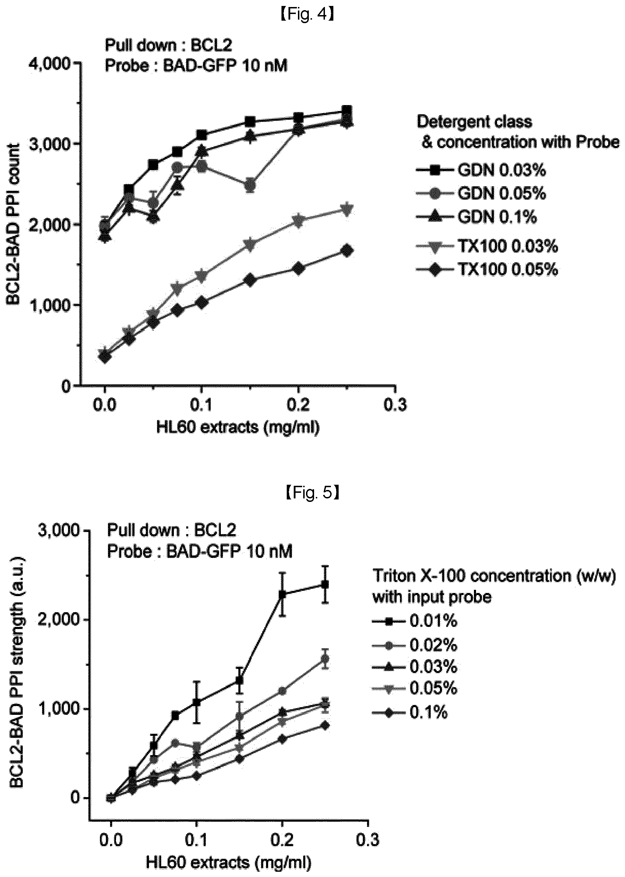

In order to identify factors that have influence on data of interaction between endogenous proteins and exogenous proteins, measurement was made of (i) PPI intensities (fluorescence intensities) according to concentrations of the analysis lysate (sample) and the probe-labeled first protein-interacting protein (FIG. 3) and (ii) PPI counts according to concentrations of the analysis lysate (sample) and kinds of the detergents contained in the cell lysate including the probe-labeled first protein-interacting protein (FIG. 4) and PPI counts according to concentrations of the detergent (FIG. 5). As a general rule, the PPI count tended to increase with the increase of concentration of the analysis lysate (sample) and the first protein-interacting protein.

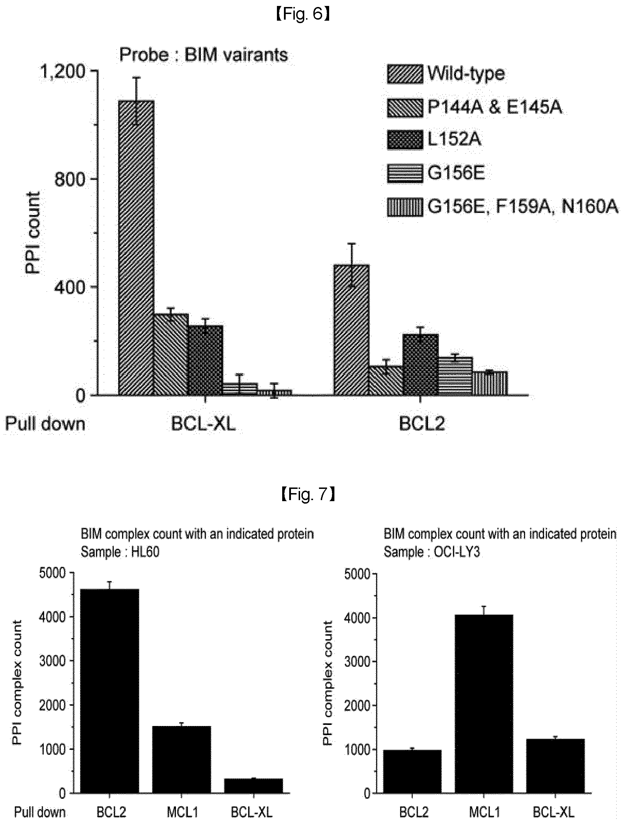

Furthermore, PPI counts were measured according to variations of the first protein-interacting proteins and are depicted in FIG. 6.

### EXAMPLE 2: Measurement of Interaction between Endogenous Proteins (Measurement of Complex)

In this Example, measurement was made of interaction between a first protein and a first protein-interacting protein, both endogenous to the same sample. In brief, the interaction was measured by detecting a first protein-first protein-interacting protein complex in which the first protein was combined with the first protein-interacting protein, in the analysis lysate. First protein-first protein-interacting protein complexes measured in this Example are listed in Table 3, below:

**TABLE 3**

| First protein | First protein-interacting (binding) protein | Related figure |
|---|---|---|
| BCL2 | BIM | FIG. 7 |
| | BAX | FIG. 8 |
| | BAK | FIG. 9 |
| BCL-XL | BIM | FIG. 7 |
| | BAD | FIG. 10 |
| | BAK | FIG. 9 |
| MCL1 | BIM | FIG. 7 |
| | NOXA | FIG. 10 |
| | BAK | FIG. 9 |

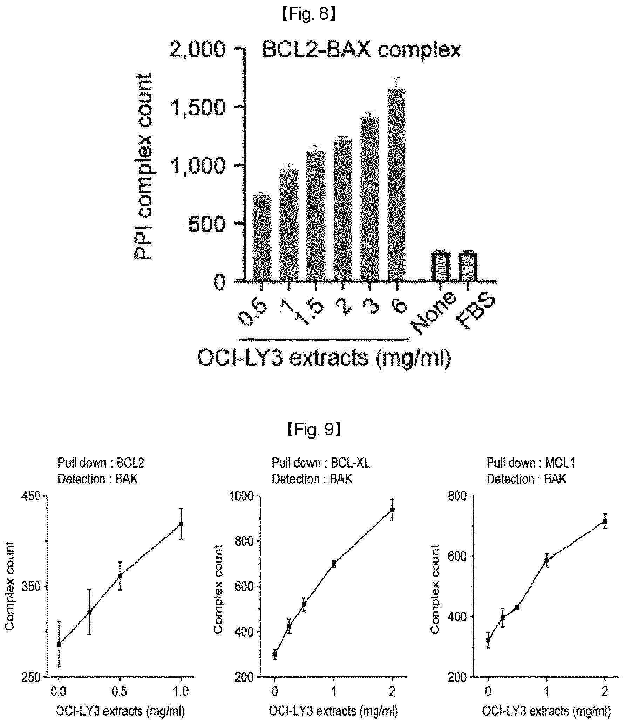

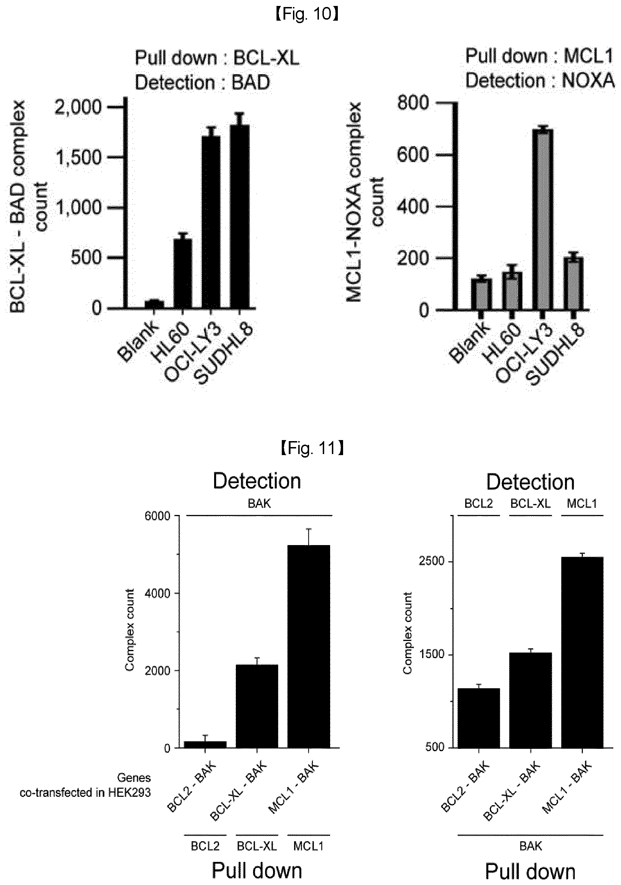

Data in the figures of Table 3 resulted from measuring the complexes formed between the first proteins pulled down to the substrate and the labeled first protein-interacting protein by means of a substance binding to the labeled first protein-interacting protein. Alternatively, complex counts formed between first protein-interacting proteins immobilized to the substrate and labeled first proteins were measured using a substance binding to the labeled first proteins (in this case, an antibody to the first protein-interacting protein and an antibody to the first protein were used as a pull-down antibody and a detecting antibody, respectively) (FIG. 11). As shown in FIGS. 7 to 11, the same detection effects could be obtained irrespective of whether the first protein or the first protein-interacting protein was pulled down to the substrate.

The results were obtained by measuring interactions between first proteins and first protein-interacting proteins, both endogenous to the sample, as follows:
First, an analysis lysate prepared in the same manner as in Example 1.1 was added to a substrate prepared with reference to Example 1.3.

As a binding substance for detecting protein-protein complexes endogenous to the sample, a detecting antibody to the first protein-interacting protein was employed. As a fluorescent labeled secondary antibody for probing the detecting antibody, Cy3 AffiniPure Donkey Anti-Mouse IgG (Jackson ImmunoResearch: 715-165-151) was prepared in a diluted form.

The detecting antibody was added to the prepared substrate. The detecting antibody was added in an amount of 10 µl to each well and incubated for 15 min at room temperature, followed by washing off excess antibodies with a washing buffer (HEPES 50mM NaCl 150mM glycerol 1% GDN 0.03%. Then, the fluorescent labeled antibody Cy3 AffiniPure Donkey Anti-Mouse IgG (Jackson ImmunoResearch: 715-165-151) was diluted 2000 folds and 10 µl of the dilution was added to each well and incubated 10 min. Excess antibodies were washed off with a washing buffer (HEPES 50mM NaCl 150mM glycerol 1% GDN 0.03%). Protein-protein interactions (complexes) were measured. The measurement was conducted in the same manner as in Example 1-4.

Information about binding substances (antibodies) used in this Example are summarized in Table 4, below:

**TABLE 4**

| First protein | Ab. Information (pull down) | First protein-interacting protein | Ab. Information (detecting) |
|---|---|---|---|
| BCL2 | #4223 (CST) | BIM | MA5-15763(Invitrogen) |
| BCL2 | | BAX | ab32503(Abcam) |
| BCL2 | | BAK | Abnova MAB8753 |
| BCL-XL | MA5-15142 (Thermo Fisher Scientific) | BIM | MA5-15763(Invitrogen) |
| BCL-XL | | BAD | 9296S (CST) |
| BCL-XL | | BAK | Abnova MAB8753 |
| MCL1 | #94296 (CST) | BIM | MA5-15763(Invitrogen) |
| MCL1 | | NOXA | ab68523(Abcam) |
| MCL1 | | BAK | Abnova MAB8753 |

As described above, the first protein and the first protein-interacting protein can be interchangeable with each other. In addition, when the antibody used is monoclonal, any equivalent clone may be used from any available manufacturers.

Thus, the antibody lists are given only to illustrate the Examples, but should not construed to limit the present disclosure.

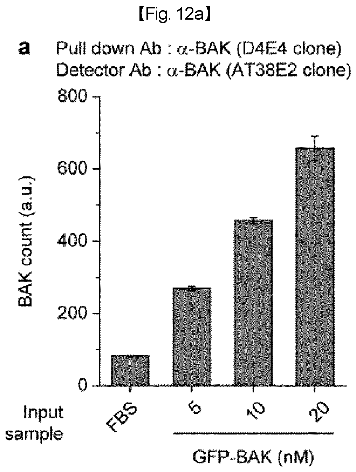

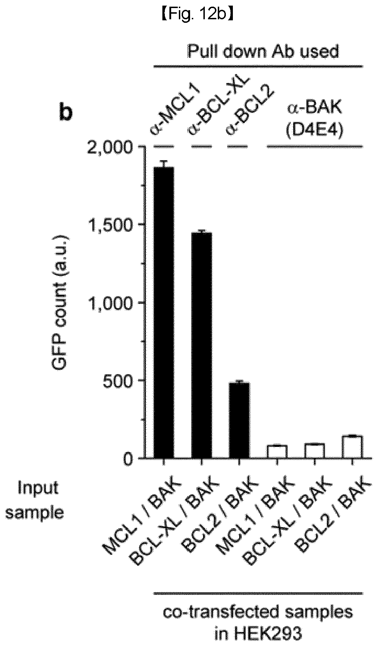

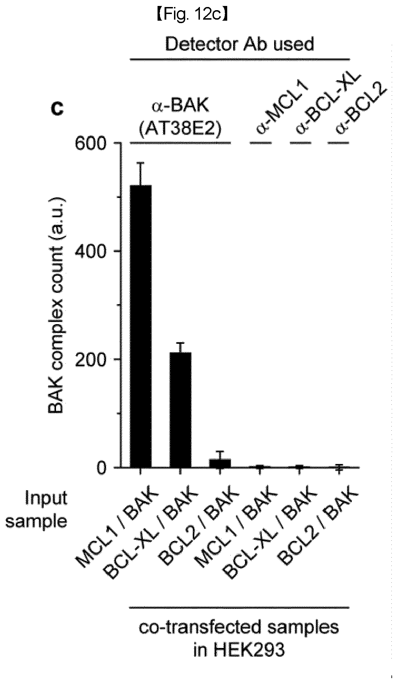

No particular limitations are imparted to the use of antibodies or binding substances that bind specifically to the first protein or the first protein-interacting protein, which form together a complex in the present disclosure. However, when sites known to be involved in the formation of a complex between two proteins are used as recognition sites for the antibodies or binding substances, detection of the complex is impossible. In an embodiment, in order to detect BCL2, BCL-XL, and MCL1, which each form a complex with BAK, it is recommended that an antibody recognizes a site other than the BH3 domain of BAK (e.g., a site of non-transmembrane domains helix 1 to helix 4, etc.) (FIG. 12).

### EXAMPLE 3. Measurement of Endogenous Protein Expression Level

In this Example, levels of specific proteins present in analysis lysates were quantitatively measured. The specific proteins included BCL2, BCL-XL, MCL1, BAX, and BAK.

In this regard, a cell lysate was prepared in the same manner as in Example 1-1 and added to a substrate prepared in the same manner as in Example 1-3. Then, the labeled detecting antibody (labeled with Cy3 AffiniPure Donkey Anti-Mouse IgG (Jackson ImmunoResearch: 715-165-151)) prepared as in Example 2 was added. Then, fluorescence signals were measured using the same method as in Example 1-4 to quantitate interactions between target proteins and detecting antibodies thereof in the analysis lysate.

In this Example, the pull-down antibody for immobilizing target proteins to a substrate and the detecting antibody for detecting the same should be identical in terms of antigen, but different in terms of binding site.

Information on the antibodies used in this Example are summarized in Table 5, below:

**TABLE 5**

| Target protein | 1^{st} binding substance (pull down) | 2^{nd} binding substance(detecting) | Related figure |
|---|---|---|---|
| BCL2 | #4223 (CST) | ab692 (Abcam) | FIG. 13 |
| BCL-XL | MA5-15142 (Thermo Fisher Scientific) | Ab77571 (Abcam) | FIG. 13 |
| MCL1 | #94296 (CST) | 14-6701-82 (Thermo) | FIG. 13 |
| BAX | sc 23959 (Santa Cruz) | Ab32503 (Abcam) | FIG. 14 |
| BAK | ab238515 (Abcam)) | AM03 (Sigma) | FIG. 14 |

The first binding substance is an antibody to be pulled down to the substrate while the second binding substance is provided to detect target proteins after the analysis lysate is added. Here, the first binding substance and the second substance may be interchangeably used. For example, the same quantitative analysis can be made even when, after pulling the second binding substance down to a substrate, the analysis lysate and the first binding substance may be provided in that order to the substrate. The given antibodies do not limit the scope of the present disclosure. However, the first and the second binding substance should avoid recognition of completely identical epitopes.

### EXAMPLE 4. Measurement of Folded Conformation-Specific Protein

This Example contemplates a method for detecting a folded conformation-specific protein present in a specimen by using an antibody or binding substance capable of specifically recognizing proteins according to folded conformations. In an embodiment, folded conformation-specific detection is applied to BAX and BAK.

It was reported that BAX protein can retain various folded conformations in cells. Of them, exposure of about 20 N-terminal amino acids is known to occur when BAX protein folds into a conformation that gives rise to apoptosis (Yi-Te Hsu et al., J. Bio. Chem. 272, (1997), Michael A. Dengler et al., Cell Rep. 27 (2019)). During migration to the mitochondrial lipid membrane, BAK protein induces apoptosis with various folded conformations (Mark Xiang Li et al., PNAS 114 (2017)). Contemplated by the present disclosure is the use of an antibody or binding substance capable of recognizing the domain that is exposed only in such specific folded conformations.

In an embodiment, an antibody known to recognize the N-terminus of BAX protein and an antibody capable of recognizing a domain exposed in any folded conformation can be used for recognizing BAX protein in an active state inducing apoptosis in a specimen. In another embodiment, an antibody selectively recognizing the BH3 domain of BAK protein and an antibody recognizing a BAK domain exposed in any conformation can be used in couple to detect BAK protein in a folded conformation allowing the exposure of the BH3 from the specimen. In Table 6 below, pairs of antibodies used to achieve the invention are listed. However, the antibodies do not limit the scope of the invention. In addition, monoclonal antibodies may be used irrespective of manufacturers.

**TABLE 6**

| Target protein | Folded conformation | Ab 1 | Ab 2 |
|---|---|---|---|
| BAX | N-terminus exposed | Ab 32503 | Sc-23959 |
| BAK | BH3 exposed | SC-517390 | SAB1305656 |

In this table, Ab 1 and Ab 2 indicate antibodies that are pulled down to the substrate and used for detection, respectively. The same results may be obtained even when the two antibodies exchange the roles with each other. For example, the same result may be expected when Ab 2 is pulled down to the substrate while Ab 1 is used as a detecting antibody.

For use in the method, an analysis lysate may be prepared in the same manner as in Example 1.

Quantitative analysis of proteins in this Example may be performed in the same manner as described in Example 2 or 3. For example, the antibodies or protein-specific binding substances used may be adapted to bind to the same antigen or target protein. Meanwhile, the two proteins or protein-specific binding substances should bind to different sites in the target protein. In addition, when two antibodies are employed, the hosts for the two antibodies are recommended to be different from each other unless the antibodies are directly labeled with corresponding probes.

This Example may be performed with reference to the method proposed in Example 2 or 3. For example, Ab 1, a specimen, and Ab 2 are added in that order to detect target proteins in specific folded conformations.

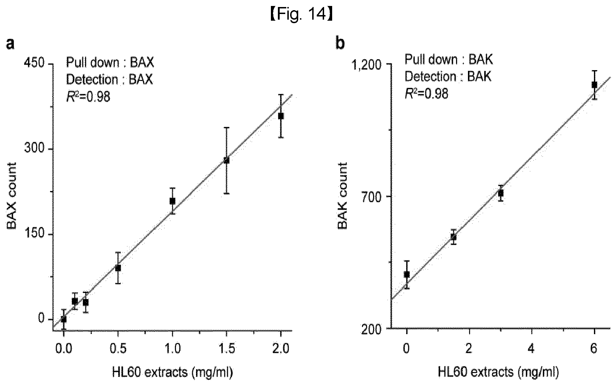

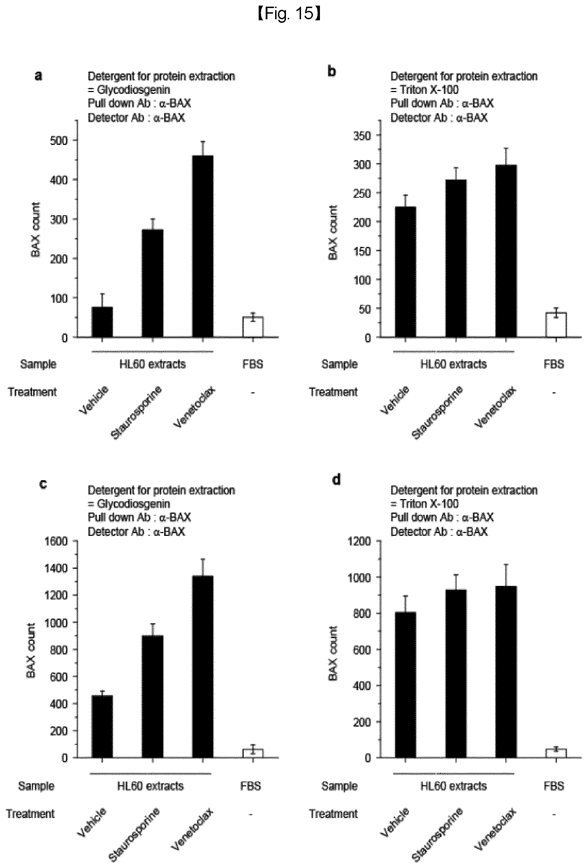

In order to achieve this Example, a specimen may be prepared by artificially inducing apoptosis. For example, cells were induced to undergo apoptosis by treatment with Staurosporine for one hour or venetoclax for 24 hours. The specimen thus prepared was lysed in a lysis buffer containing GDN to afford an analysis lysate which was then observed to exhibit increased signals when N-terminus exposed BAX was detected therefrom (FIG. 14). For an analysis lysate prepared from the same specimen using a lysis buffer containing Triton X-100, N-terminal exposure may be forcibly induced by the non-ionic detergent Triton X-100. From this analysis lysate, N-terminus-exposed BAX was detected (FIG. 15). After an analysis lysate was prepared from an apoptosis-induced specimen using GDN, signals were increased when the N-terminus-exposed BAX was measured. In contrast, when artificial N-terminus exposure was made using Triton X-100, constant values were obtained irrespective of apoptosis induction.

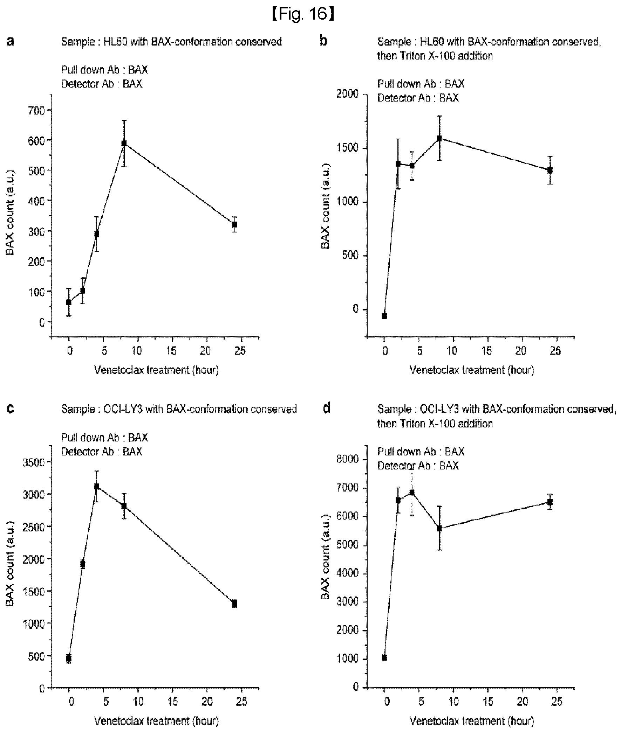

In another embodiment, apoptosis was induced by incubating the AML cell line HL60 or the DLBCL cell line OCI-LY3 with venetoclax and ratios of the N-terminus exposure of BAX were measured. After treatment with 100 nM venetoclax for 2, 4, 8, or 24 hrs, the specimen was lysed with a lysis buffer containing GDN to prepare an analysis lysate. Then, BAX detection signals changed with time of treatment with venetoclax (FIG. 16). In contrast, after N-terminus exposure was induced by further mixing the same analysis lysate with Triton X-100 at a final concentration of 1%, the BAX amount was observed to undergo almost no changes with time.

As verified above, the folded conformation-specific protein detection method provided herein can provide indices helpful for analyzing cell states, for example, a degree of apoptosis.

### EXAMPLE 5. Measurement of Change in Intracellular BCL2 Family Signaling Pathway with Treatment with BCL2 Family Protein-Targeting Inhibitor

This Example contemplates a method for measuring a change in intracellular signal transduction with treatment with BCL2 family protein-targeting inhibitors. More specifically, provided is a method for comprehensively analyzing intensities of inter- and intra-sample protein-protein interaction (complex) for various BCL2 family proteins (e.g., BCL2, BCL-XL, MCL1) and changes in protein expression levels, in which the proteins were treated with the BCL2-targeting inhibitor venetoclax, or the MCL1-targeting inhibiting AZD5991.

In order to achieve the goal of this Example, the inter-sample protein-protein interaction, the intra-sample protein-protein interaction, and changes in expression levels may be measured in the same manners as in Examples 1, 2, and 3 and 4, respectively.

For example, the targeting inhibitor venetoclax or AZD5991 may be applied at a concentration of 100 nM to cells for 24 hrs. For a control, DMSO may be applied under the same condition.

Analysis contents in this Example are summarized in Tables 7 and 8, below:

**TABLE 7**

| Analysis of Inter-Sample Protein-Protein Interaction | |
|---|---|
| First protein | First protein-interacting protein |
| BCL2 | BAD-GFP |
| | BIM*-GFP |
| BCL-XL | BAD-GFP |
| | BIM*-GFP |
| MCL1 | GFP-NOXA |
| | BIM*-GFP |

(In Table 7, BIM* stands for GFP-labeled BH3 domain excised from BIM, and the first protein is endogenous to the analysis lysate and pulled down to the surface of the substrate)

**TABLE 8**

| Measurements of Intra-Sample Protein Complexes | |
|---|---|
| First protein | First protein-interacting (binding) protein |
| BCL2 | BIM |
| | BAX |
| | BAK |
| BCL-XL | BIM |
| | BAD |
| | BAK |
| MCL1 | BIM |
| | NOXA |
| | BAK |

In Table 8, the first proteins and the first protein-interacting (binding) protein, which form complexes, are both contained in the analysis lysates. The same result may be obtained even if either of the two proteins is pulled down to the surface of the substrate. For example, the antibody pulled down to the substrate surface may be an antibody against the first protein or the first protein-interacting (binding) protein.

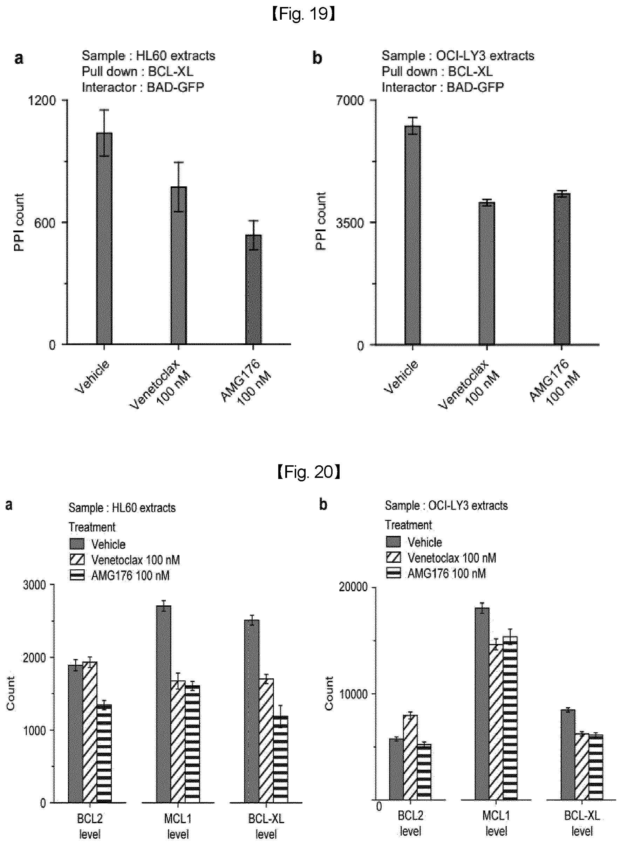

For example, the method proposed in this Example can quantitatively measure changes in PPI intensity and complex formation after and before treatment with BCL2-targeting inhibitors. To this end, HL60 or OCI-LY3 cells were treated with DMSO, venetoclax 100 nM (BCL2-targeting inhibitor), or AMG176 100 nM (MCL1-targeting inhibitor) for 24 hours, after which analysis lysates were prepared from the respective specimens and measured for the indices given above. Treatment with venetoclax was observed to increase BCL2-BAD PPI intensity in both HL60 and OCI-LY3 (FIG. 17). In contrast, treatment with the MCL1-targeting inhibitor AMG176 did not change BCL2-BAD PPI. The result was not limited to venetoclax-BCL2 only. Upon treatment with AMG176, MCL1-NOXA PPI was observed to selectively increase (FIG. 18). BCL-XL was responsive to none of venetoclax/AMG176 and PPI intensity varied depending on BCL-XL expression levels. These data indicate that BCL2- and MCL1-targeting inhibitors selectively generate PPI intensities of corresponding proteins (FIGS. 19 and 20).

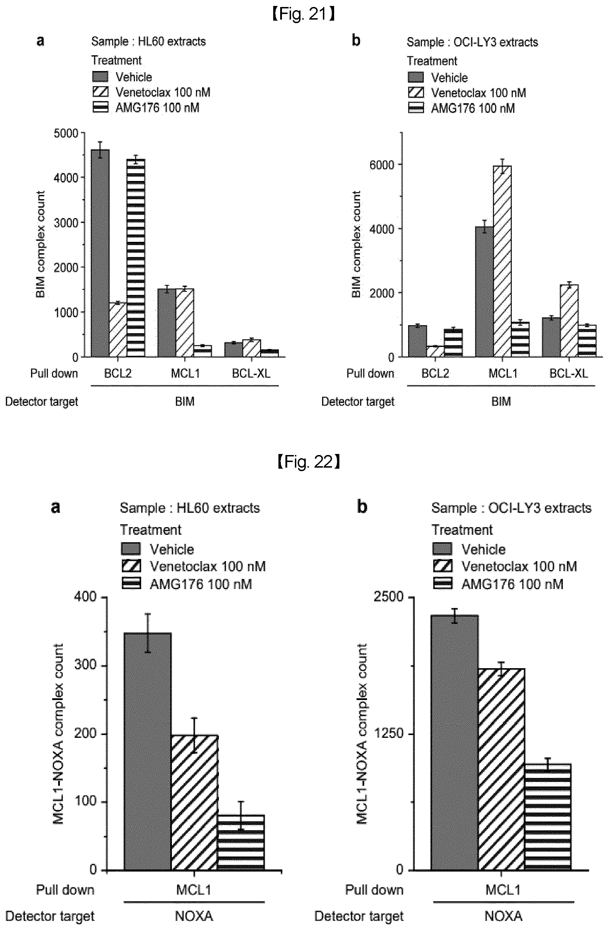

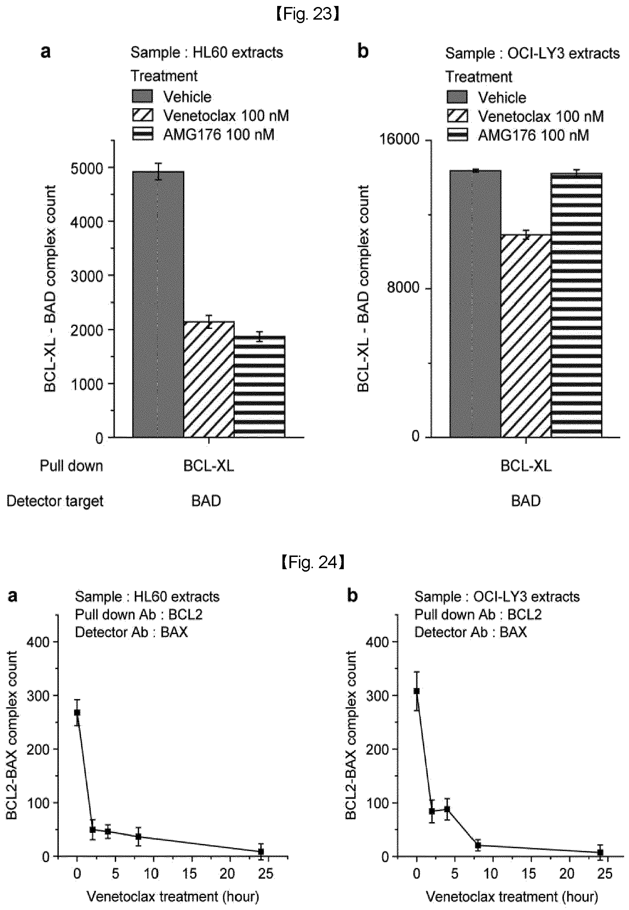

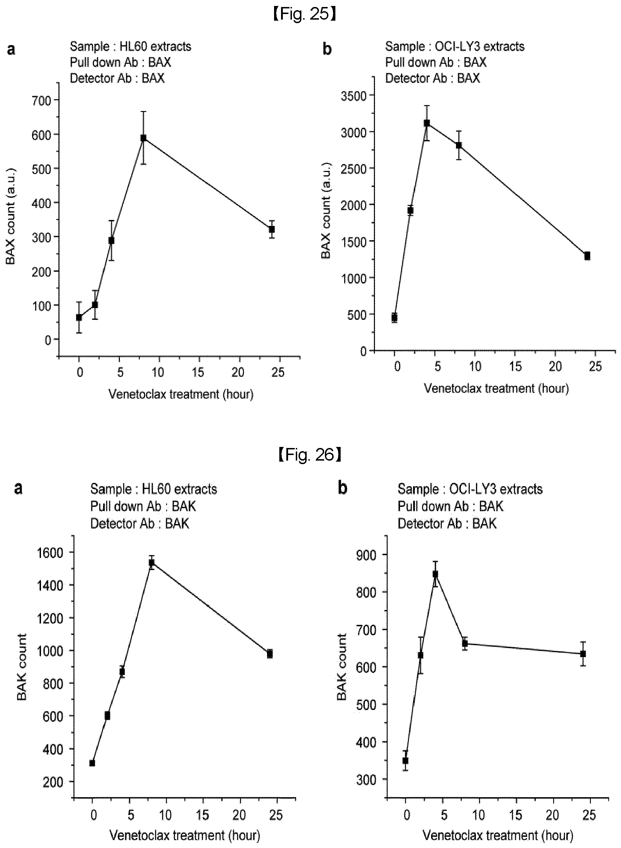

In another embodiment, changes in complex formation were measured according to treatment with BCL2-targeting inhibitors. Under the same conditions as for the PPI measurement, the harvested specimens were analyzed to measure changes in the formation of BCL2 family-BIM complexes (FIG. 21), MCL1-NOXA complex (FIG. 22), and BCLXL-BAD complex (FIG. 23). Moreover, the method can be applied to the measurement of changes in BCL2-BAX complex (FIG. 24) and expression levels of BAX and BAK (FIGS. 25 and 26).

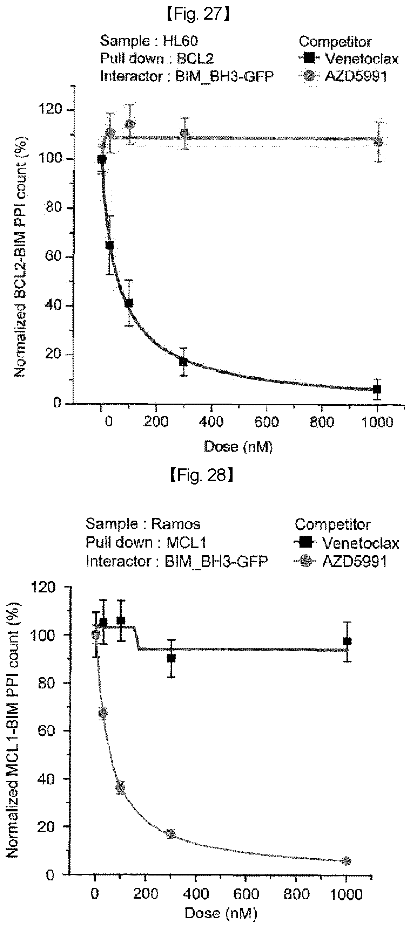

This Example can be used to measure direct relationship between targeting inhibitors used and the BCL2 family proteins and related signaling proteins. To this end, BCL2 or MCL1 protein was used as a first protein while BIM-GFP protein served as a first protein-interacting protein. While the first protein was pulled down to the surface of the substrate, various concentrations of the targeting inhibitor venetoclax or AZD5991 (MCL1-targeting inhibitor) was added, together with the first protein-interacting protein, to measure PPI intensity changes. The BCL2-BIM PPI intensity decreased only in the presence of the BCL2-targeting inhibitor venetoclax, but was not affected by AZD5991 (FIG. 27). In contrast, when MCL1 served as a first protein, only AZD5991 decreased the MCL1-BIM PPI intensity (FIG. 28).

Taken together, the results indicate that the method proposed herein can quantitatively analyze BCL2 family protein characteristics (PPI intensity, complex formation, expression levels, etc.) that appear with the application of BCL2 family-targeting inhibitors to specimens. In addition, changes in PPI intensity were identified to be directly controlled by the application of the drugs. Consequently, the method provided herein is designed to measure changes of BCL2 family proteins according to treatment with BCL2 targeting inhibitors and can be used as a molecular diagnosis technique for specimens.

### EXAMPLE 6. Practical Analysis Method for Patient Specimen

The method illustrated in this Example is to measure characteristics of BCL2 family proteins in a specimen from a patient (e.g., acute myeloid leukemia (AML) or breast cancer).

For equivalent comparison between patient specimens, total protein concentrations may be determined using at least one selected from Bradford assay, Lowry assay, and infrared absorption. Quantitative comparison between specimens is allowed under the condition of maintaining constant concentrations of specimens added to the substrate. For AML patients, specimens may be isolated from the blood or bone marrow. From solid cancer tissues, specimens may be obtained by surgery or needle biopsy.

All the assays proposed in Examples 1, 2, and 3 are possible for patient specimens, but without limitations thereto. The assays are all applicable to both blood cancer and solid cancer.

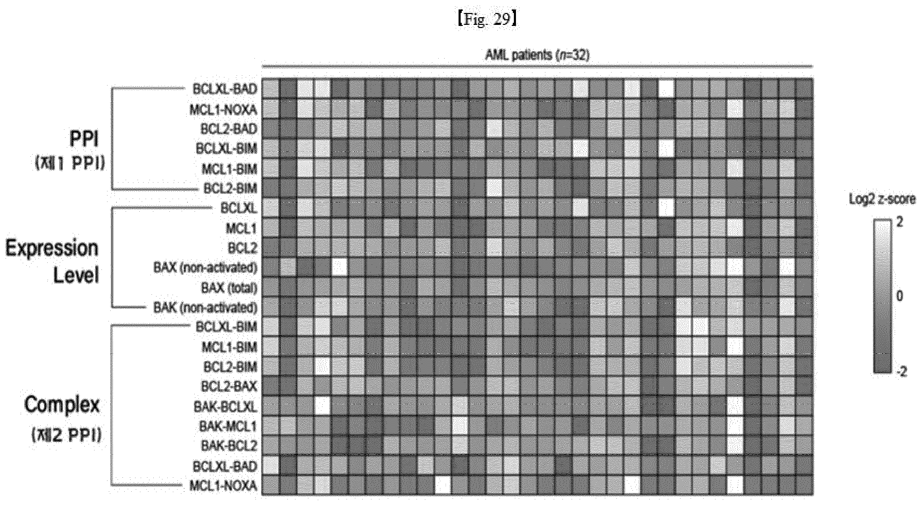

For example, based on analysis results of specimens acquired from 32 AML blood cancer patients, an analysis method for comprehensively assaying BCL2 family proteins for PPI (Example 1), complex (Example 2), and expression level (see Example 3) can be provided (FIG. 29).

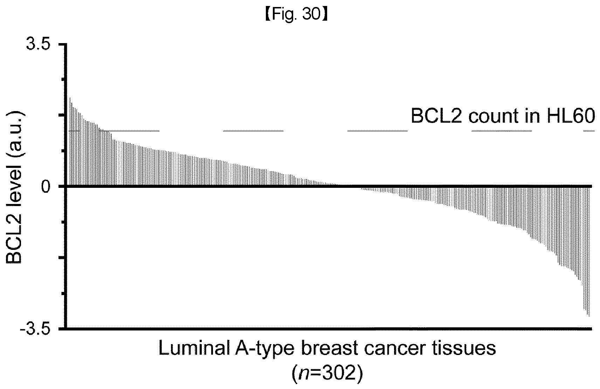

In another embodiment, the analysis of specimens acquired from 302 breast cancer patients can be used to quantitate expression levels of BCL2 proteins endogenous to the specimen (FIG. 30).

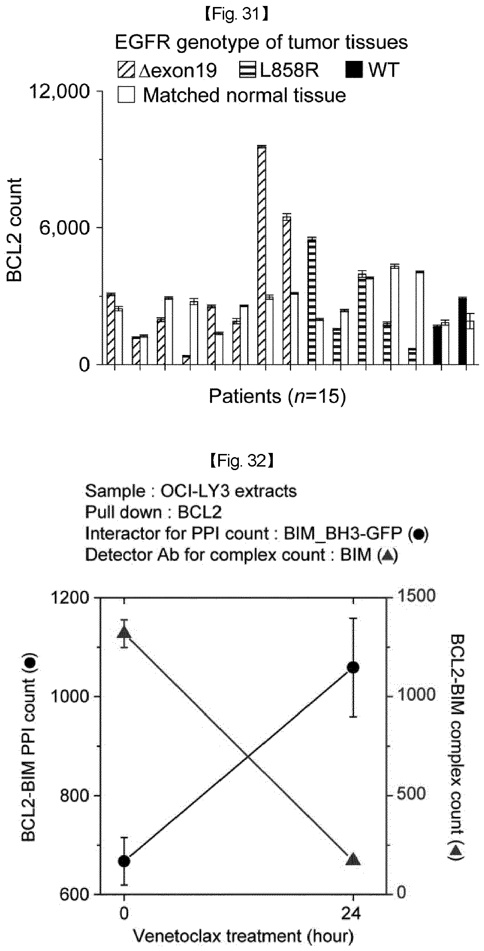

In another embodiment, the method based on data of specimens from 15 lung adenocarcinoma patients can be applied to the quantitation of BCL2 protein expression levels (FIG. 31).

### EXAMPLE 7. Comparison of Interaction Measurement of Example 1 and Complex Measurement of Example 2

The two methods proposed herein, that is, the measurement of PPI intensity by using a first protein-interacting protein (Example 1) and the measurement of a complex between a first protein-interacting protein and a first protein-binding protein (Example 2) can provide different information.

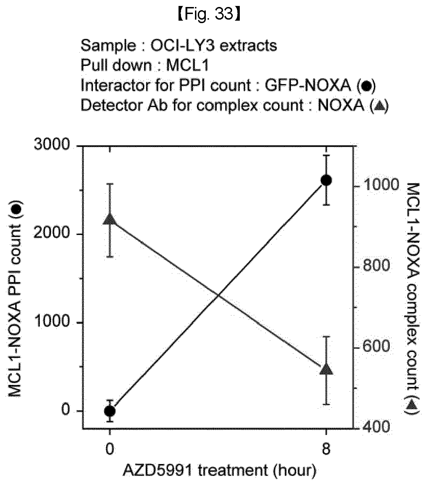

Briefly, the methods can detect a difference in PPI intensity and complex formation before and after incubation of OCI-LY3 cells with Venetoclax 100 nM or AZD5991 100 nM. For example, specimens treated with venetoclax were observed to increase BCL2-BIM PPI counts, but decrease BCL2-BIM complex counts (FIG. 32). In addition, specimens treated with AZD5991 increased MCL1-NOXA PPI counts, but decreased MCL1-NOXA complex counts (FIG. 33). As is understood from the assays, the PPI count measurement achieved as in Example 1 is basically different from the complex count measurement achieved in Example 2.

### EXAMPLE 8. Method for Determining Drug Administration by Tracing Patient State through Observation of Change in BCL2 Family Protein Interaction

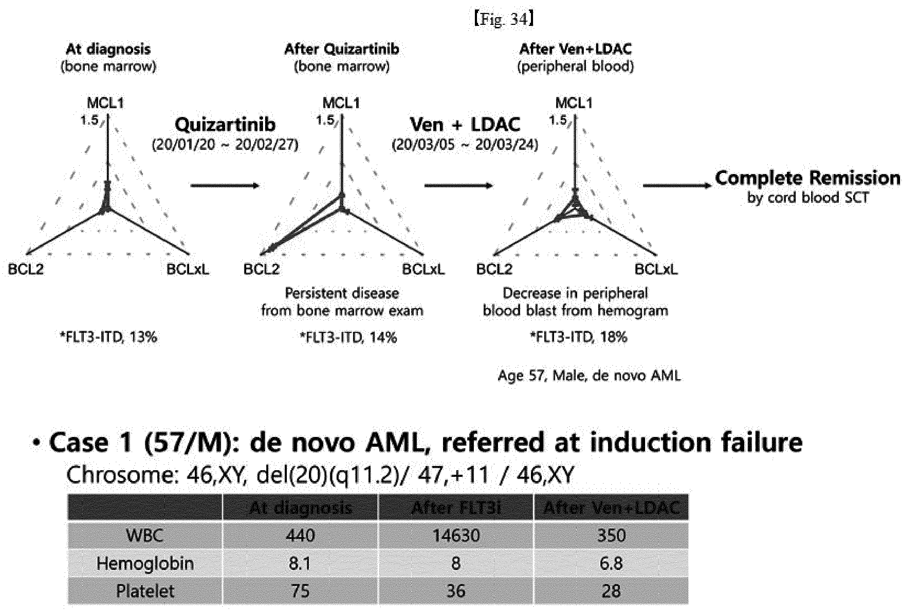

The method provided herein can lead to a method for tracing molecular states of a patient with time. In greater detail, the interaction analysis of bone marrow or blood specimens collected at various stages from a blood cancer patient can be used to provide information helpful in determining BCL2 family protein-dependence and whether to administer a corresponding targeted anticancer agent. In this regard, for patient 1, i) PPI assay between each of BCL2, MCL1, and BCL-XL, and BIM-GFP in the bone marrow extracted at the time of diagnosis is made; ii) the same assay is performed in the bone marrow extracted after treatment with the FLT3-targeting anticancer agent quizartinib; and iii) the same assay is performed for the whole blood specimen acquired after venetoclax and Low-dose cytarabine (LDAC) is prescribed based on PPI information acquired in step ii) (FIG. 34). At the time of diagnosis, there was an insufficient ground for prescribing venetoclax because the patient showed a low BCL2-BIM PPI. After prescription of the FLT3 targeting inhibitor quizartinib, however, signal rewiring occurred to drastic increase the BCL2-BIM PPI. When treated with a combination of venetoclax and LDAC due to the phenomenon, the patient was observed to decrease again in BCL2-BIM PPI. In a clinical opinion, there was a drastic rise in leukocyte count after quizartinib prescription, but treatment with venetoclax and LDAC in combination reduced the leukocyte count to a stable level, demonstrating the medicinal efficacy of venetoclax even at a clinical level. Afterwards, a cord blood stem cell transplantation brought about complete remission in the patient.

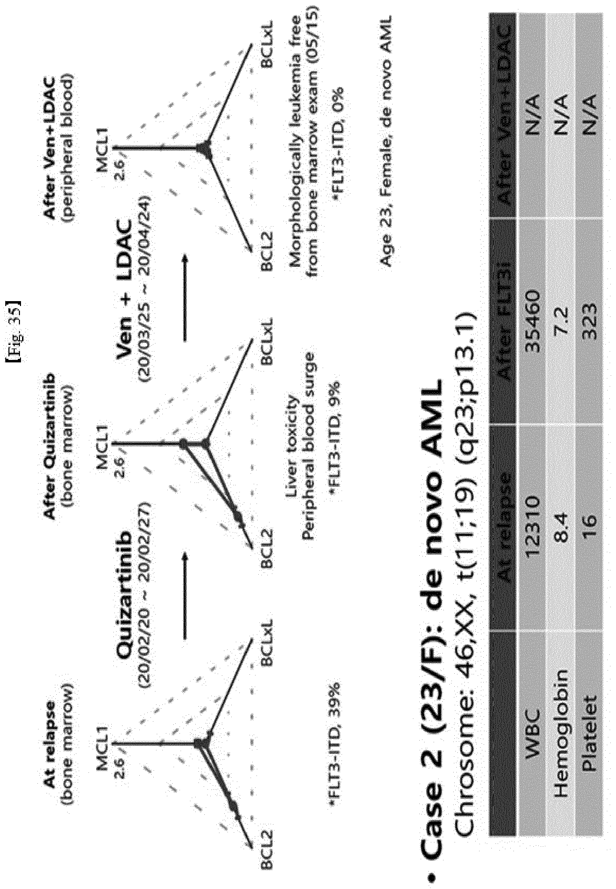

In addition, time series diagnosis was performed for patient 2 in the same manner as for patient 1 (FIG. 35). In patient 2, the signaling pathway of the BCL2 family protein could not be controlled by prescribing the FLT3 targeting inhibitor, either. After treatment of a combination of venetoclax and LDAC, however, the BCL2-BIM PPI was observed to drastically decrease. Consistent with this result, a leukocyte count test showed that the leukocyte count was about three folds increased after quizartinib prescription. From the results obtained above, it is understood that quizartinib prescription cannot bring about any clinical effect.

### EXAMPLE 9. Development of Method for Prediction of Responsiveness to BCL2 Targeting Inhibitor

Through the method proposed herein, responsiveness of an acute myeloid leukemia patient to the BCL2 targeting inhibitor venetoclax can be predicted.

Briefly, among the BCL2 family proteins in specimens (bone marrow or blood) extracted as in Example 6 from AML patients, six was measured for PPI count (Example 1), six for expression level (Example 3), and nine for complex count (Example 2) (see Table 9, below).

**TABLE 9**

| Analysis for | Measurement | Analysis for | Measurement |
|---|---|---|---|
| PPI (see Example 1) | BCLXL-BAD PPI | Complex (see Example 2) | BCLXL-BIM CPX |
| | MCL1-NOXA PPI | | MCL1-BIM CPX |
| | BCL2-BAD PPI | | BCL2-BIM CPX |
| | BCLXL-BIM (BH3) PPI | | BCL2-BAX CPX |
| | MCL1-BIM (BH3) PPI | | BAK-BCLXL CPX |
| | BCL2-BIM (BH3) PPI | | BAK-MCL1 CPX |
| Expression level (see Example 3) | BCL-XL Lv | | BAK-BCL2 CPX |
| | MCL1 Lv | | BCLXL-BAD CPX |
| | BCL2 Lv | | MCL1-NOXA CPX |
| | BAX LV (-TX100) | | |
| | BAX LV (+TX100) | | |
| | BAK LV (Y164) | | |

For measurement of responsiveness to venetoclax, viable cells were counted by flow cytometry after specimens isolated from patients were treated with 0, 30, 100, 300, or 1000 nM of venetoclax for 24 hrs. Dose response curves were drawn for remaining viable cell counts. The responsiveness of individual patients to venetoclax was quantitated using inverse values of AUC (area under the curve).

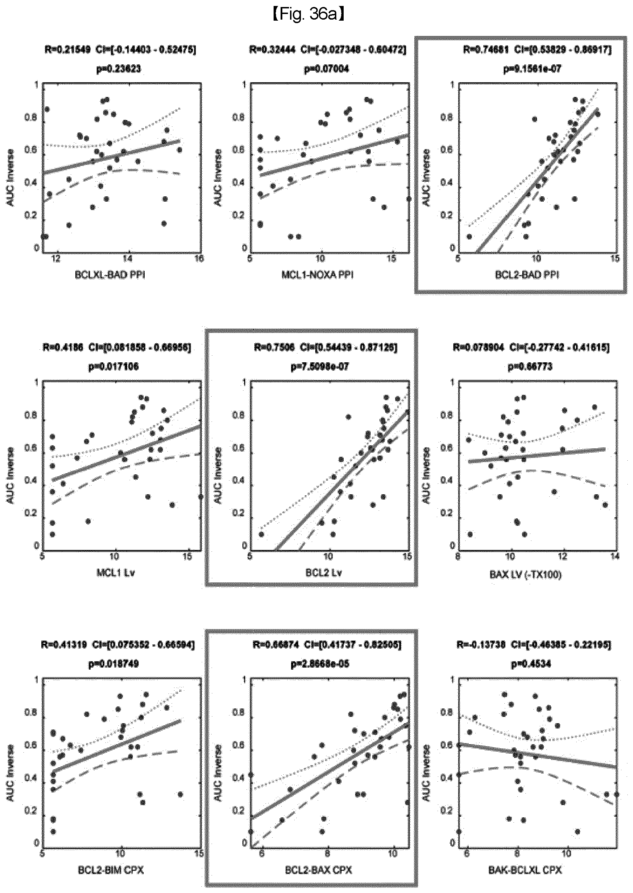

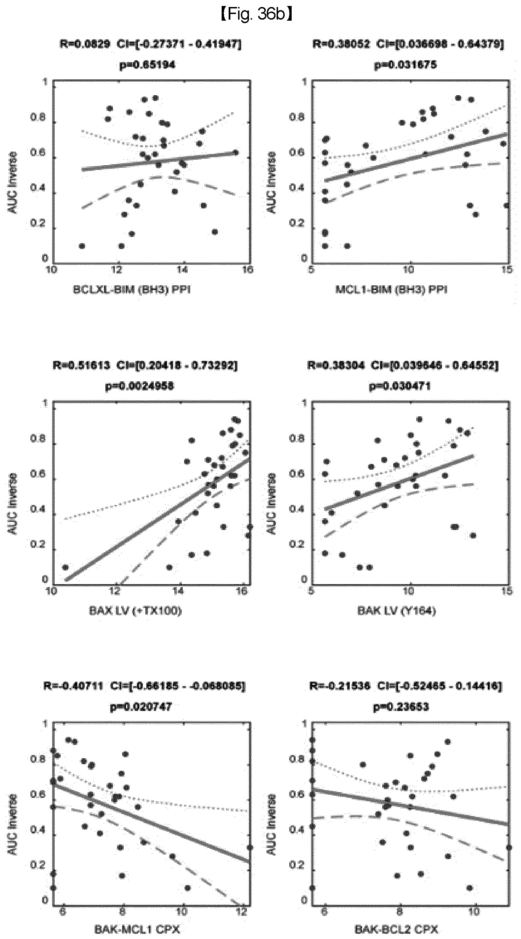

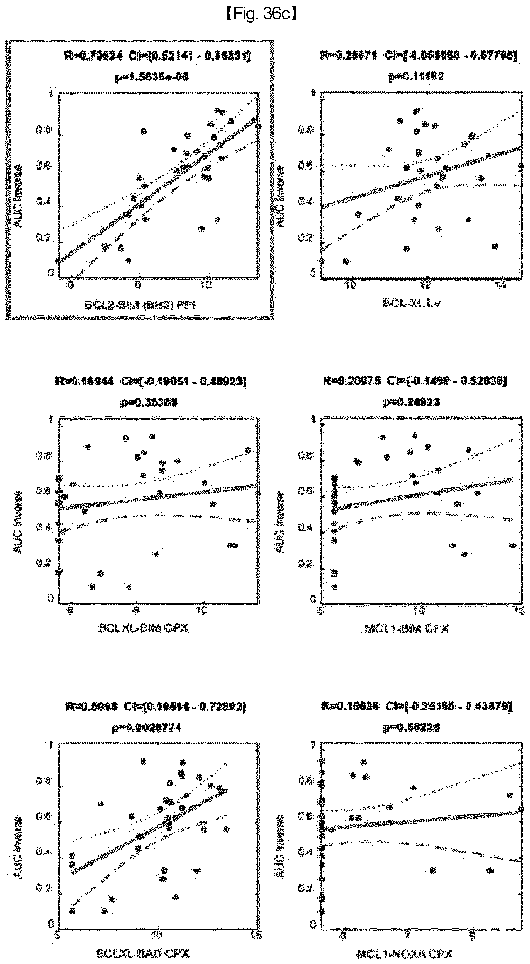

Through linear correlation analysis for venetoclax responsiveness to individual measurements, the highest predictive indices were acquired (FIGS. 36a-36c). For quantitative comparison, a linear correlation coefficient (R, Pearson's correlation coefficient) was acquired. Among a total of 21 indices, BCL2-BAD PPI, BCL2-BIM PPI, BCL2 expression level, and BCL2-BAX complex count were measured to have R values of 0.65 or greater (four indices indicated by squares in FIGS. 36a-36c). However, the selected indices do not limit the scope of the invention (e.g., kinds of indices involved in simulated models) in establishing a venetoclax response predicting model.

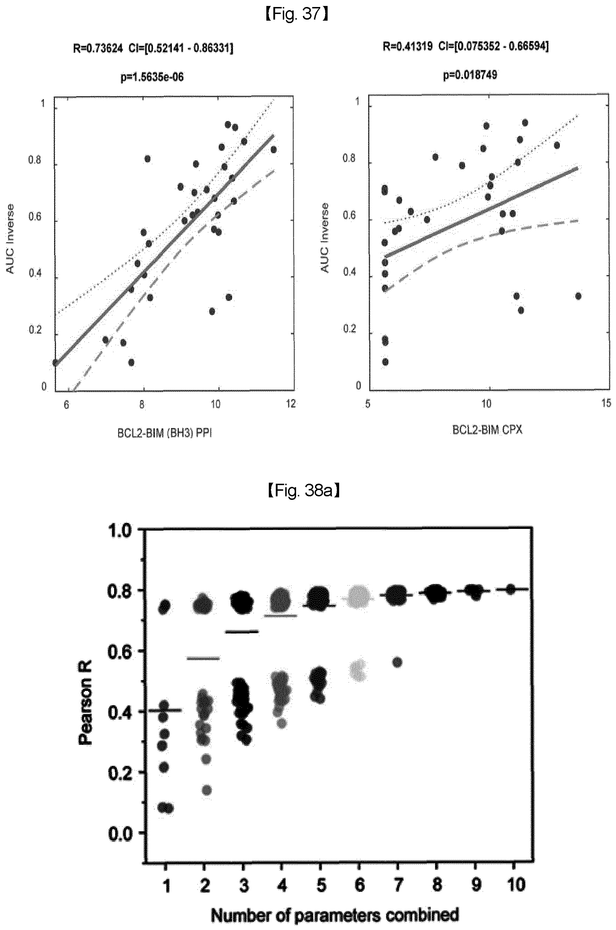

In order to confirm that PPI counts and complex counts are indices different from each other, BCL2-BIM PPI counts and BCL2-BIM complex counts can be each compared with responsiveness to venetoclax (FIG. 37). The correlation was observed to be as high as R=0.74 between BCL2-BIM PPI and venetoclax, but decrease to R=0.41 for BCL2-BIM complex count. These results account for use of PPI counts and complex counts as different indices, demonstrating that PPI counts allow the highly accurate prediction for responsiveness to venetoclax.

Combinations of the measurements can raise the accuracy of prediction for responsiveness to venetoclax. Such combinations of the measurements can be achieved using various methods including multiple linear regression, logistic regression, support vector machine, etc. In this Example, multiple linear regression was employed, but without limitations to the scope of the present disclosure. When a single parameter (index) was employed, the highest value was detected at about R=0.75, but increased as the number of parameters increased (FIG. 38a).

As many as 2,097,151 combinations can be elicited from 21 parameters (indices). Various parameters such as biological plausibility, mathematic plausibility, etc. may be considered for searching for optimal models.

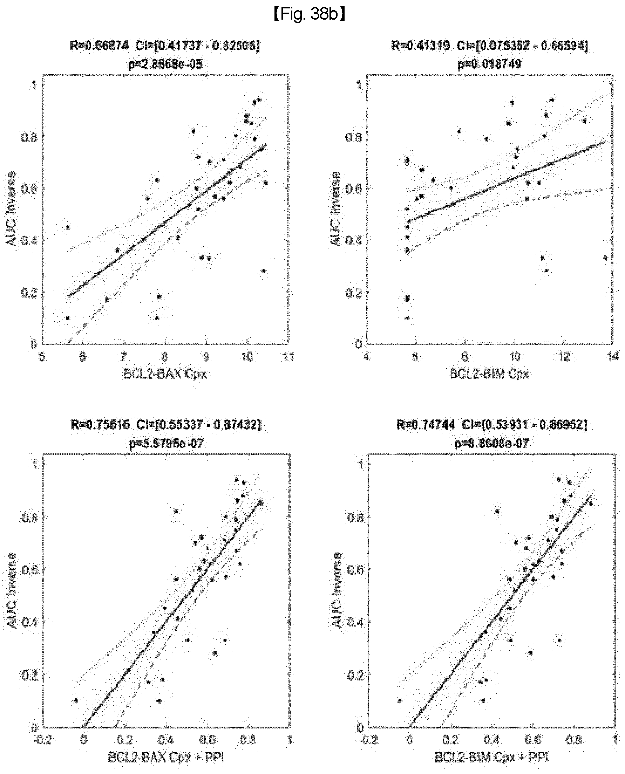

For example, in order to demonstrate that a combination of two or more indices achieves better analysis results than a single indices, correlations between BCL2-BAX or BCL2-BIM complexes and responsiveness to venetoclax and between a combination of BCL2-BAX or BCL2-BIM complex and BCL2-BAD PPI and responsiveness to venetoclax are depicted in the upper and lower panels of FIG. 38b, respectively. As shown in FIG. 38b, R values were measured to be 0.67 and 0.41 for BCL2-BAX and BCL2-BIM complexes, respectively, but increased to 0.76 and 0.75 when the complexes were combined with BCL2-BAD PPI, indicating that combinations of two indices lead to more improved prediction results for drug responsiveness.

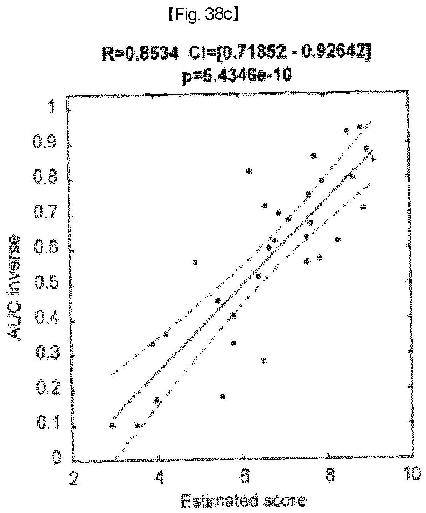

By way of example, the linear correlation coefficient R was observed to be 0.85 in a model in which a total of 5 parameters were combined (FIG. 38c). In this regard, the parameters used are given in Table 10, below. This combination exhibited the most significant results among combinations of various parameters.

**TABLE 10**

| Parameter | Measurement |
|---|---|
| PPI | BCLXL-BAD PPI |
| Expression level | BAX LV (+TX100) |
| Complex | BCL2-BAX CPX |
| | BAK-BCLXL CPX |
| | BAK-MCL1 CPX |

### EXAMPLE 10. Establishment of Method for Predicting Responsiveness to MCL1-Targeting Inhibitor

A method for predicting responsiveness to a MCL1-targeting inhibitor can be provided in the same manner as in Example 9. For example, AZD5991 is used as a MCL1-targeting inhibitor, but so long as it retains a similar mechanism of action, any targeting inhibitor may be equally used. Kinds of the drugs used do not limit the scope of the invention.

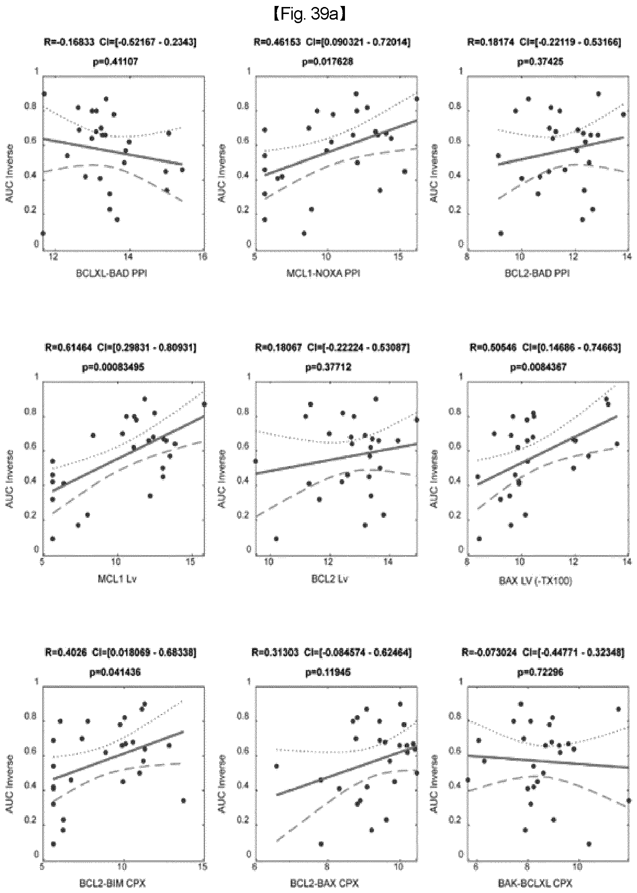

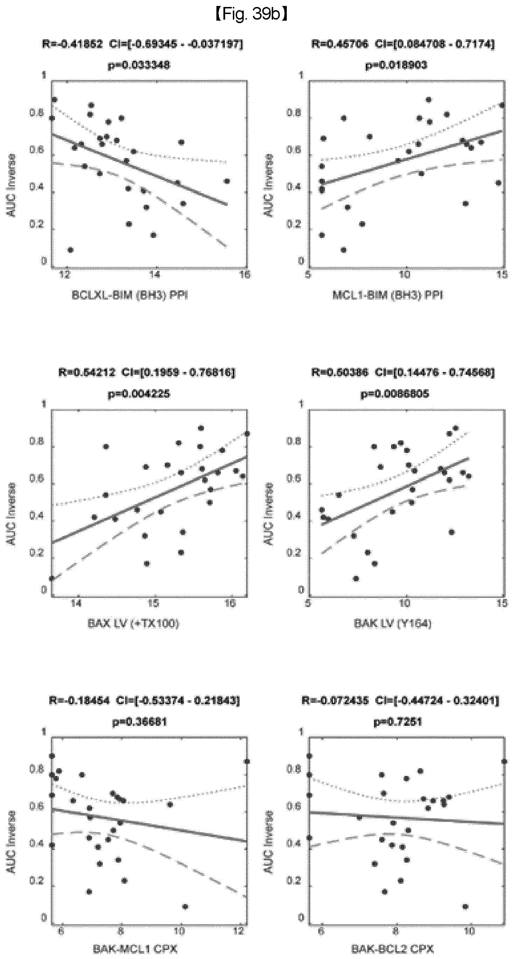

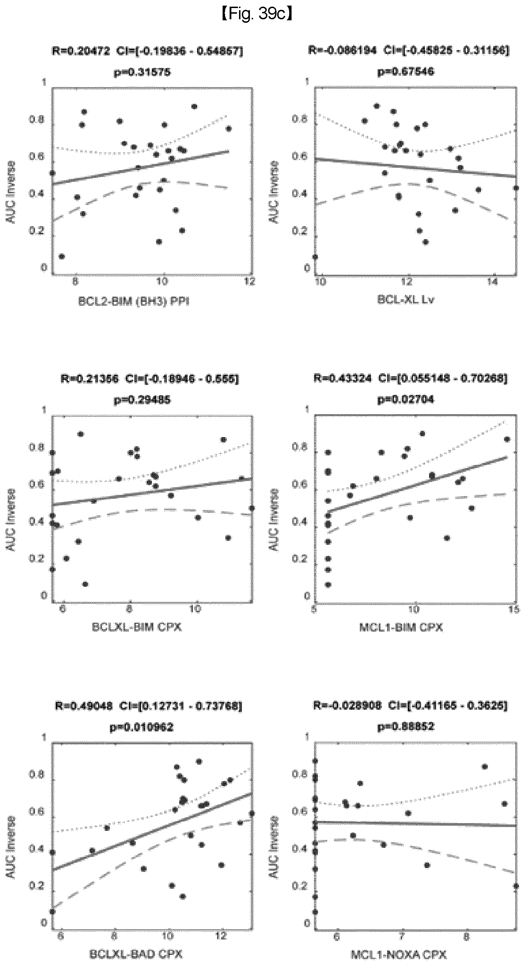

Linear correlation analysis may be performed for single indices as in Example 9, revealing that the indices including MCL1-BIM PPI, BCLXL-BIM PPI, BAX expression level, and BAK expression level correlate with responsiveness to AZD5991 (FIGS. 39a-39c).

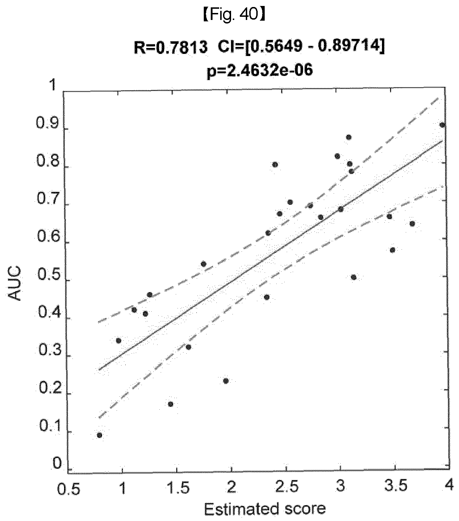

In order to increase the accuracy of responsiveness prediction, advantage may be taken of combinations of various parameters as in Example 9. By way of example, correlation analysis for responsiveness to AZD5991 with combinations of the parameters listed in Table 11, below, acquired R=0.78 (FIG. 40), exhibiting the most significant results among combinations of various parameters.

**TABLE 11**

| Parameter | Measurement |
|---|---|
| PPI | BCLXL-BIM PPI |
| | MCL1-BIM PPI |
| Expression level | BAX (+TX100) level |
| Complex | BAK-BCLXL CPX |

It will be appreciated by those having ordinary knowledge in the art to which the present invention belongs that the present invention may be practiced in other specific forms without changing the technical spirit and essential features of the present invention. Therefore, it should be understood that the above-described embodiments are illustrative but not restrictive in all aspects.

## Claims

1. A method for prediction of a response of a subject to a BCL2 family protein-targeting drug, the method comprising at least one selected from the following steps of:
(i) measuring interaction between a first protein within a sample and a first protein-interacting protein, the first protein-interacting protein being provided externally;
(ii) measuring interaction between a first protein endogenous to a sample and a first protein-interacting protein, the first protein-interacting protein being endogenous to the sample; and
(iii) measuring a level of the first protein, the first protein-interacting protein, or both thereof in the sample,
wherein,
the first protein is at least one selected from anti-apoptotic proteins of BCL2 family proteins and the first protein-interacting protein is at least one selected from pro-apoptotic proteins of BCL2 family proteins, or the first protein is at least one selected from pro-apoptotic proteins of BCL2 family proteins and the first protein-interacting protein is at least one selected from anti-apoptotic proteins of BCL2 family proteins, and
the sample includes a cell or cell lysate isolated from the subject.

2. The method of claim 1, wherein
the first protein is at least one selected from the group consisting of BCL2, BCLxL, BCLw, BFL1, and MCL1, and the first protein-interacting protein is at least one selected from the group consisting of BAD, BIM, NOXA, PUMA, BMF, tBID, BIK, HRK, BAX, BAK, and BOK, or
the first protein is at least one selected from the group consisting of BAD, BIM, NOXA, PUMA, BMF, tBID, BIK, HRK, BAX, BAK, and BOK and the first protein-interacting protein is at least one selected from the group consisting of BCL2, BCLxL, BCLw, BFL1, and MCL1.

3. The method of claim 1, wherein
step (i) comprises the sub-steps of bringing the externally provided first protein-interacting protein labeled with a probe into contact with the first protein immobilized to a substrate and measuring protein-protein interaction counts between the first protein within the sample and the externally provided first protein-interacting protein;
step (ii) comprises the sub-steps of bringing the sample is brought into contact with a substrate including a substance that binds to the first protein and measuring a level of a complex formed between the first protein and the first protein-interacting protein, both endogenous to the sample; and
step (iii) comprises a sub-step of measuring a total expression level of either or both of the first protein and the first protein-interacting protein, both endogenous to the sample.

4. The method of claim 3, wherein the sub-step of measuring protein-protein interaction in step (i) comprises:
(1) providing the sample to a substrate including a substance binding to the first protein to immobilize the first protein to the substrate;
(2-1) externally providing a probe-labeled first protein-interacting protein to the substrate to allow a reaction with the first protein on the substrate; and
(3) measuring a signal generated by the probe within a near field region on the surface of the substrate using a total internal reflection fluorescence (TIRF) microscope.

5. The method of claim 2, wherein step (i) comprises at least one selected from the following sub-steps of:
(i-1) measuring protein-protein interaction between BCL2 protein within a sample and externally provided BAD protein;
(i-2) measuring protein-protein interaction between BCL2 protein within a sample and externally provided BIM protein;
(i-3) measuring protein-protein interaction between BCL2 protein within a sample and externally provided BAX protein;
(i-4) measuring protein-protein interaction between BCL2 protein within a sample and externally provided BAK protein;
(i-5) measuring protein-protein interaction between BCLxl protein within a sample and externally provided BAD protein;
(i-6) measuring protein-protein interaction between BCLxl protein within a sample and externally provided BIM protein;
(i-7) measuring protein-protein interaction between BCLxl protein within a sample and externally provided BAX protein;
(i-8) measuring protein-protein interaction between BCLxl protein within a sample and externally provided BAK protein;
(i-9) measuring protein-protein interaction between MCL1 protein within a sample and externally provided NOXA protein;
(i-10) measuring protein-protein interaction between MCL1 protein within a sample and externally provided BIM protein;
(i-11) measuring protein-protein interaction between MCL1 protein within a sample and externally provided BAX protein; and
(i-12) measuring protein-protein interaction between MCL1 protein within a sample and externally provided BAK protein.

6. The method of claim 3, wherein the sub-step of measuring protein-protein interaction in step (ii) comprises:
(1) providing a sample to a substrate including a substance binding to the first protein to immobilize the first protein to the substrate;
(2-2) providing the substrate with a probe-labeled substance that binds to the first protein-interacting protein to allow a reaction with the first protein-interacting protein of a complex formed between the first protein endogenous to the sample and the first protein-interacting protein exogenous to the sample; and
(3) measuring a signal generated by the probe within a near field region on the surface of the substrate using a total internal reflection fluorescence (TIRF) microscope, or
(1') providing a sample to a substrate including a substance binding to the first protein-interacting protein to immobilize the first protein-interacting protein to the substrate;
(2'-2) providing the substrate with a probe-labeled substance that binds to the first protein to allow a reaction with the first protein of a complex formed between the first protein endogenous to the sample and the first protein-interacting protein endogenous to the sample; and
(3') measuring a signal generated by the probe within a near field region on the surface of the substrate using a total internal reflection fluorescence (TIRF) microscope.

7. The method of claim 2, wherein step (ii) comprises at least one selected from the following sub-steps of:
(ii-1) measuring a level of a complex formed between BCL2 protein within a sample and BIM protein within a sample;
(ii-2) measuring a level of a complex formed between BCL2 protein within a sample and BAX protein within a sample;
(ii-3) measuring a level of a complex formed between BCL2 protein within a sample and BAK protein within a sample;
(ii-4) measuring a level of a complex formed between BCLxl protein within a sample and BAD protein within a sample;
(ii-5) measuring a level of a complex formed between BCLxl protein within a sample and BIM protein within a sample;
(ii-6) measuring a level of a complex formed between BCLxl protein within a sample and BAX protein within a sample;
(ii-7) measuring a level of a complex formed between BCLxl protein within a sample and BAK protein within a sample;
(ii-8) measuring a level of a complex formed between MCL1 protein within a sample and NOXA protein within a sample;
(ii-9) measuring a level of a complex formed between MCL1 protein within a sample and BIM protein within a sample;
(ii-10) measuring a level of a complex formed between MCL1 protein within a sample and BAX protein within a sample; and
(ii-11) measuring a level of a complex formed between MCL1 protein within a sample and BAK protein within a sample.

8. The method of claim 3, wherein the measuring sub-step in step (iii) comprises:
providing a sample to a substrate including either or both of a substance for capturing the first protein and a substance for capturing the first protein-interacting protein to immobilize either or both of the first protein and the first protein-interacting protein to the substrate;
providing either or both of a probe-labeled substance binding to the first protein and a probe-labeled substance binding to the first protein-interacting protein to the substrate to allow a reaction; and
measuring a signal generated by the probe within a near field region on the surface of the substrate using a total internal reflection fluorescence (TIRF) microscope.

9. The method of claim 2, wherein step (iii) comprises at least one of the following sub-steps of:
(iii-1) measuring a level of BCL2 protein within the sample;
(iii-2) measuring a level of BCLxl protein within the sample;
(iii-3) measuring a level of MCL1 protein within the sample;
(iii-4) measuring a level of BAX protein within the sample; and
(iii-5) measuring a level of BAK protein within the sample.

10. The method of claim 2, comprising at least one selected from:
(i-1) measuring protein-protein interaction between BCL2 protein within a sample and externally provided BAD protein;
(i-2) measuring protein-protein interaction between BCL2 protein within a sample and externally provided BIM protein;
(i-3) measuring protein-protein interaction between BCL2 protein within a sample and externally provided BAX protein;
(i-4) measuring protein-protein interaction between BCL2 protein within a sample and externally provided BAK protein;
(i-5) measuring protein-protein interaction between BCLxl protein within a sample and externally provided BAD protein;
(i-6) measuring protein-protein interaction between BCLxl protein within a sample and externally provided BIM protein;
(i-7) measuring protein-protein interaction between BCLxl protein within a sample and externally provided BAX protein;
(i-8) measuring protein-protein interaction between BCLxl protein within a sample and externally provided BAK protein;
(i-9) measuring protein-protein interaction between MCL1 protein within a sample and externally provided NOXA protein;
(i-10) measuring protein-protein interaction between MCL1 protein within a sample and externally provided BIM protein;
(i-11) measuring protein-protein interaction between MCL1 protein within a sample and externally provided BAX protein;
(i-12) measuring protein-protein interaction between MCL1 protein within a sample and externally provided BAK protein;
(ii-1) measuring a level of a complex formed between BCL2 protein within a sample and BIM protein within a sample;
(ii-2) measuring a level of a complex formed between BCL2 protein within a sample and BAX protein within a sample;
(ii-3) measuring a level of a complex formed between BCL2 protein within a sample and BAK protein within a sample;
(ii-4) measuring a level of a complex formed between BCLxl protein within a sample and BAD protein within a sample;
(ii-5) measuring a level of a complex formed between BCLxl protein within a sample and BIM protein within a sample;
(ii-6) measuring a level of a complex formed between BCLxl protein within a sample and BAX protein within a sample;
(ii-7) measuring a level of a complex formed between BCLxl protein within a sample and BAK protein within a sample;
(ii-8) measuring a level of a complex formed between MCL1 protein within a sample and NOXA protein within a sample;
(ii-9) measuring a level of a complex formed between MCL1 protein within a sample and BIM protein within a sample;
(ii-10) measuring a level of a complex formed between MCL1 protein within a sample and BAX protein within a sample;
(ii-11) measuring a level of a complex formed between MCL1 protein within a sample and BAK protein within a sample;
(iii-1) measuring a level of BCL2 protein within the sample;
(iii-2) measuring a level of BCLxl protein within the sample;
(iii-3) measuring a level of MCL1 protein within the sample;
(iii-4) measuring a level of BAX protein within the sample; and
(iii-5) measuring a level of BAK protein within the sample.

11. The method of claim 10, comprising
at least one selected from the following steps (i-1) to (i-12) and at least one selected from steps (ii-1) to (ii-11),
at least one selected from the following steps (i-1) to (i-12) and at least one selected from the following steps (iii-1) to (iii-5),
at least one selected from the following steps (ii-1) to (ii-11) and at least one selected from the following steps (iii-1) to (iii-5), or
at least one selected from the following steps (i-1) to (i-12), at least one selected from the following steps (ii-1) to (ii-11), and at least one selected from the following steps (iii-1) to (iii-5):
(i-1) measuring protein-protein interaction between BCL2 protein within a sample and externally provided BAD protein;
(i-2) measuring protein-protein interaction between BCL2 protein within a sample and externally provided BIM protein;
(i-3) measuring protein-protein interaction between BCL2 protein within a sample and externally provided BAX protein;
(i-4) measuring protein-protein interaction between BCL2 protein within a sample and externally provided BAK protein;
(i-5) measuring protein-protein interaction between BCLxl protein within a sample and externally provided BAD protein;
(i-6) measuring protein-protein interaction between BCLxl protein within a sample and externally provided BIM protein;
(i-7) measuring protein-protein interaction between BCLxl protein within a sample and externally provided BAX protein;
(i-8) measuring protein-protein interaction between BCLxl protein within a sample and externally provided BAK protein;
(i-9) measuring protein-protein interaction between MCL1 protein within a sample and externally provided NOXA protein;
(i-10) measuring protein-protein interaction between MCL1 protein within a sample and externally provided BIM protein;
(i-11) measuring protein-protein interaction between MCL1 protein within a sample and externally provided BAX protein;
(i-12) measuring protein-protein interaction between MCL1 protein within a sample and externally provided BAK protein;
(ii-1) measuring a level of a complex formed between BCL2 protein within a sample and BIM protein within a sample;
(ii-2) measuring a level of a complex formed between BCL2 protein within a sample and BAX protein within a sample;
(ii-3) measuring a level of a complex formed between BCL2 protein within a sample and BAK protein within a sample;
(ii-4) measuring a level of a complex formed between BCLxl protein within a sample and BAD protein within a sample;
(ii-5) measuring a level of a complex formed between BCLxl protein within a sample and BIM protein within a sample;
(ii-6) measuring a level of a complex formed between BCLxl protein within a sample and BAX protein within a sample;
(ii-7) measuring a level of a complex formed between BCLxl protein within a sample and BAK protein within a sample;
(ii-8) measuring a level of a complex formed between MCL1 protein within a sample and NOXA protein within a sample;
(ii-9) measuring a level of a complex formed between MCL1 protein within a sample and BIM protein within a sample;
(ii-10) measuring a level of a complex formed between MCL1 protein within a sample and BAX protein within a sample;
(ii-11) measuring a level of a complex formed between MCL1 protein within a sample and BAK protein within a sample;
(iii-1) measuring a level of BCL2 protein within the sample;
(iii-2) measuring a level of BCLxl protein within the sample;
(iii-3) measuring a level of MCL1 protein within the sample;
(iii-4) measuring a level of BAX protein within the sample; and
(iii-5) measuring a level of BAK protein within the sample.

12. The method of claim 1, wherein the BCL2 family protein-targeting drug is a BCL2 inhibitor selected from the group consisting of venetoclax, ABT737, and an anti-BCL2 antibody.

13. The method of claim 1, wherein the BCL2 family protein-targeting drug is a MCL1 inhibitor selected from the group consisting of an anti-MCL-1 antibody, AMG-176, AZD5991, and Maritoclax.

14. The method of claim 1, wherein the BCL2 family protein-targeting drug is a BCLxL inhibitor selected from the group consisting of an anti-BCLxL antibody and ABT263.

15. The method of claim 1, wherein the subject is a cancer patient and the cell is a cancer cell isolated from the subject.

16. The method of claim 1, wherein the cancer is a blood cancer.
